(19) Europäisches Patentamt
European Patent Office
Office européen des brevets

(11) EP 1 076 657 B1

(12) EUROPÄISCHE PATENTSCHRIFT

(45) Veröffentlichungstag und Bekanntmachung des Hinweises auf die Patenterteilung:
**04.08.2004 Patentblatt 2004/32**

(51) Int Cl.[7]: **C07D 401/12**, A61K 31/40, C07D 209/22, C07D 209/24, A61P 11/00

(21) Anmeldenummer: **99920779.8**

(22) Anmeldetag: **24.04.1999**

(86) Internationale Anmeldenummer:
**PCT/EP1999/002792**

(87) Internationale Veröffentlichungsnummer:
**WO 1999/055696 (04.11.1999 Gazette 1999/44)**

# (54) NEUE HYDROXYINDOLE, DEREN VERWENDUNG ALS INHIBITOREN DER PHOSPHODIESTERASE 4 UND VERFAHREN ZU DEREN HERSTELLUNG

NEW HYDROXYINDOLES, THEIR USE AS PHOSPHODIESTERASE 4 INHIBITORS AND METHOD FOR PRODUCING SAME

NOUVEAUX HYDROXYINDOLES, LEUR UTILISATION COMME INHIBITEURS DE LA PHOSPHODIESTERASE 4 ET LEUR PROCEDE DE PREPARATION

(84) Benannte Vertragsstaaten:
**AT BE CH CY DE DK ES FI FR GB GR IE IT LI LU MC NL PT SE**
Benannte Erstreckungsstaaten:
**LT LV MK RO SI**

(30) Priorität: **28.04.1998 DE 19818964**
**17.04.1999 DE 19917504**

(43) Veröffentlichungstag der Anmeldung:
**21.02.2001 Patentblatt 2001/08**

(73) Patentinhaber: **elbion AG**
**01445 Radebeul (DE)**

(72) Erfinder:
- **HÖFGEN, Norbert**
  **D-01458 Ottendorf-Okrilla (DE)**
- **EGERLAND, Ute**
  **D-01445 Radebeul (DE)**
- **POPPE, Hildegard**
  **D-01109 Dresden (DE)**
- **MARX, Degenhard**
  **D-01796 Pirna (DE)**
- **SZELENYI, Stefan**
  **D-90571 Schwaig (DE)**
- **KRONBACH, Thomas**
  **D-01445 Radebeul (DE)**
- **POLYMEROPOULOS, Emmanuel**
  **D-60325 Frankfurt (DE)**
- **HEER, Sabine**
  **D-01445 Radebeul (DE)**

(74) Vertreter: **Böhm, Brigitte, Dipl.-Chem. Dr. et al**
**Weickmann & Weickmann**
**Patentanwälte**
**Postfach 86 08 20**
**81635 München (DE)**

(56) Entgegenhaltungen:
**EP-A- 0 490 263**
**WO-A-97/23457**
**WO-A-98/09946**

- **ROBERT D. DILLARD ET AL.: "Indole inhibitors of human nonpancreatic secretory phospholipase A2. 1. Indole-3-acetamides" JOURNAL OF MEDICINAL CHEMISTRY., Bd. 39, Nr. 26, - 1996 Seiten 5119-5136, XP002113652 AMERICAN CHEMICAL SOCIETY. WASHINGTON., US ISSN: 0022-2623**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Technisches Gebiet

Die Erfindung betrifft substituierte Hydroxyindole der allgemeinen Formel 1 ,

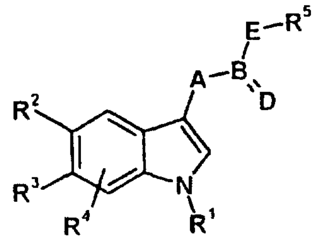

1

Verfahren zu deren Herstellung, pharmazeutische Zubereitungen, die diese Verbindungen enthalten sowie die pharmazeutische Verwendung dieser Verbindungen, die Inhibitoren der Phosphodiesterase 4 sind, als Wirkstoffe zur Behandlung von Erkrankungen, die mit einer Hemmung der Phosphodiesterase 4 - Aktivität in immunkompetenten Zellen (z.B Makrophagen und Lymphozyten) durch die erfindungsgemäßen Verbindungen zu beeinflussen sind.

Stand der Technik

Die Aktivierung von Rezeptoren der Zellmembran durch Transmitter führt zur Aktivierung des "second messenger"-Systems Die Adenylatcyclase synthetisiert aus AMP und GMP das wirksame cyclische AMP (cAMP) bzw. cyclische GMP (cGMP) Diese fuhren z.B in glatten Muskelzellen zur Erschlaffung bzw in Entzündungszellen zur Hemmung der Mediatorfreisetzung bzw -synthese Der Abbau der "second messenger" cAMP und cGMP erfolgt durch die Phosphodiesterasen (PDE) Bisher sind 7 Familien von PDE-Enzymen (PDE1-7) bekannt, die sich durch ihre Substratspezifität (cAMP, cGMP oder beides) und die Abhängigkeit von anderen Substraten (z B Calmodulin) unterscheiden. Diese Isoenzyme besitzen unterschiedliche Funktionen im Körper und sind in den einzelnen Zellarten unterschiedlich ausgeprägt (Beavo JA, Conti M and Heaslip RJ. Multiple cyclic nucleotide phosphodiesterases. Mol. Pharmacol. 1994, 46:399-405; Hall IP. Isoenzyme selective phosphodiesterase inhibitors: potential clinical uses, Br. J. clin. Pharmacol. 1993, 35-1-7). Durch Hemmung der verschiedenen PDE Isoenzymtypen kommt es zu einer Kumulation von cAMP bzw. cGMP in den Zellen, was therapeutisch genutzt werden kann (Torphy TJ, Livi GP, Christensen SB Novel Phosphodiesterase Inhibitors for the Therapy of Asthma, Drug News and Perspectives 1993, 6:203-214).
In den für allergische Entzündungen wichtigen Zellen (Lymphozyten, Mastzellen, eosinophile Granulozyten, Makrophagen) ist das vorherrschende PDE-Isoenzym der Typ 4 (Torphy, J T and Undem, B J Phosphordiesterase inhibitors new opportunities for the treatment of asthma. Thorax 1991, 46:512-523) Die Hemmung der PDE 4 durch geeignete Inhibitoren wird daher als wichtiger Ansatz zur Therapie einer Vielzahl allergisch induzierter Erkrankungen betrachtet (Schudt Ch, Dent G, Rabe K Phosphodiesterase Inhibitors, Academic Press London 1996).
Eine wichtige Eigenschaft von Phosphodiesterase 4 Inhibitoren ist die Hemmung der Freisetzung von Tumornekrosefaktor a (TNF$\alpha$) aus Entzündungszellen. TNF$\alpha$ ist ein bedeutendes pro-inflammatorisches Cytokin, das eine Vielzahl biologischer Prozesse beeinflußt. Freigesetzt wird TNF$\alpha$ zum Beispiel aus aktivierten Macrophagen, aktivierten T-Lymphozyten, Mastzellen, Basophilen, Fibroblasten, Endothelzellen und Astrozyten im Gehirn. Es wirkt selbst aktivierend auf Neutrophile, Eosinophile, Fibroblasten und Endothelzellen, wodurch verschiedene gewebezerstörende Mediatoren freigesetzt werden. In Monozyten, Macrophagen und T-Lymphozyten bewirkt TNF$\alpha$ die vermehrte Produktion von weiteren proinflammatorischen Cytokinen wie GM-CSF (Granulocy-macrophage colony-stimulating factor) oder Interleukin-8. Auf Grund seiner entzundungsfördernden und katabolischen Wirkung spielt TNF$\alpha$ bei einer Vielzahl von Erkrankungen, wie Entzündungen der Atemwege, Entzündungen der Gelenke, endotoxischer Schock. Gewebsabstoßungen. AIDS und zahlreichen anderen immunologischen Erkrankungen eine zentrale Rolle Für die Therapie solcher mit TNF$\alpha$ verbundener Erkrankungen sind Inhibitoren der Phosphodiesterase 4 somit ebenfalls geeignet.
Chronisch obstruktive Lungenerkrankungen (chronic obstructive pulmonary diseases, COPD) sind in der Bevölkerung weit verbreitet und und haben auch eine große ökonomische Bedeutung. So verursachen COPD-Erkrankungen ca. 10-15 % aller Krankheitskosten in den entwickelten Ländern und ca. 25 % aller Todesfälle in den USA sind auf diese Ursache zurückzuführen (Norman P.. COPD: New developments and therapeutic opportunities, Drug News Perspect. 11 (7), 431-437, 1998), allerdings sind die Patienten zum Todeszeitpunkt meist über 55 Jahre alt (Nolte D.:

Chronische Bronchitis - eine Volkskrankheit multifaktorieller Genese. Atemw.-Lungenkrkh. 20 (5), 260-267, 1994). Die WHO schätzt ein, das COPD innerhalb der nächsten 20 Jahren die dritthäufigste Todesursache sein wird.

Unter dem Krankheitsbild der chronisch obstruktiven Lungenerkrankungen (COPD) werden verschiedene Krankheitsbilder von chronischen Bronchitiden mit den Symptomen Husten und Auswurf sowie fortschreitender und irreversiblen Verschlechterung der Lungenfunktion (besonders betroffen ist die Exspiration) zusammengefaßt. Der Krankheitsverlauf ist schubförmig und oft durch bakterielle Infektionen kompliziert (Rennard S. I. COPD: Overview of definitions, Epidemiology, and factors influencing its development Chest, 113 (4) Suppl., 235S-241S, 1998) Im Verlauf der Erkrankung nimmt die Lungenfunktion stetig ab, die Lunge wird zunehmend emphysematös und die Atemnot der Patienten wird offensichtlich. Diese Erkrankung beeinträchtigt deutlich die Lebensqualität der Patienten (Kurzatmigkeit, geringe Belastbarkeit) und verkürzt signifikant deren Lebenserwartung. Der Hauptrisikofaktor neben Umweltfaktoren ist das Rauchen (Kummer F.: Asthma und COPD. Atemw -Lungenkrkh. 20 (5), 299-302, 1994; Rennard S. I.: COPD: Overview of definitions, Epidemiology, and factors influencing its development. Chest, 113 (4) Suppl., 235S-241S, 1998 ) und daher sind Männer deutlich häufiger betroffen, als Frauen. Durch die Veränderung der Lebensgewohnheiten und den Anstieg der Anzahl der Raucherinnen wird sich dieses Bild jedoch in Zukunft verschieben.

Die gegenwärtige Therapie zielt nur auf die Linderung der Symptome, ohne ursächlich in die Progression der Erkrankung einzugreifen. Der Einsatz von langwirkenden Beta2-Agonisten (z B Salmeterol) evtl. in Kombination mit muscarinergen Antagonisten (z B Ipratropium) verbessert die Lungenfunktion durch Bronchodilatation und wird routinemäßig eingesetzt (Norman P.: COPD: New developments and therapeutic opportunities, Drug News Perspect. 11 (7), 431-437, 1998). Eine große Rolle bei den COPD-Schüben spielen bakterielle Infektionen, die mit Antibiotika behandelt werden müssen (Wilson R.: The role of infection in COPD, Chest, 113 (4) Suppl., 242S-248S, 1998; Grossman R. F. : The value of antibiotics and the outcomes of antibiotic therapy in exacerbations of COPD. Chest, 113 (4) Suppl., 249S-255S, 1998). Die Therapie dieser Erkrankung ist bisher noch unbefriedigend, besonders im Hinblick auf die stetige Abnahme der Lungenfunktion. Neue Therapieansätze, die an Entzündungsmediatoren, Proteasen oder Adhäsionsmolekülen angreifen, könnten sehr erfolgversprechend sein (Barnes P.J.: Chronic obstructive disease: new opportunities for drug development, TiPS 10 (19), 415-423, 1998).

Unabhängig von den die Erkrankung komplizierenden bakteriellen Infektionen, findet man in den Bronchien eine chronische Entzündung, welche durch neutrophile Granulozyten dominiert wird. Für die beobachteten strukturellen Veränderungen in den Atemwegen (Emphysem) werden unter anderem die durch neutrophile Granulozyten freigesetzten Mediatoren und Enzyme verantwortlich gemacht. Die Hemmung der Aktivität der neutrophilen Granulozyten ist somit ein rationaler Ansatz, um ein Fortschreiten der COPD (Verschlechterung der Lungenfunktionparameter) zu verhindern oder zu verlangsamen. Ein wichtiger Stimulus für die Aktivierung der Granulozyten ist das pro-inflammatorische Cytokin TNF$\alpha$ (tumour necrosis factor). So ist bekannt, daß TNF$\alpha$ die Bildung von Sauerstoff-Radikalen durch neutrophile Granulozyten stimuliert (Jersmann, H.P.A.; Rathjen, D.A. and Ferrante A.: Enhancement of LPS-induced neutrophil oxygen radical production by TNF$\alpha$, Infection and Immumty, 4, 1744-1747, 1998). PDE4-Inhibitoren können sehr wirksam die Freisetzung von TNF$\alpha$ aus einer Vielzahl von Zellen hemmen und somit die Aktivität der neutrophilen Granulozyten unterdrucken. Der unspezifische PDE-Inhibitor Pentoxifylline ist in der Lage, sowohl die Bildung von Sauerstoff-Radikalen als auch die Phagozytosefähigkeit von neutrophilen Granulozyten zu hemmen (Wenisch. C.: Zedtwitz-Liebenstein. K.: Parschalk. B. and Graninger W.: Effect of pentoxifylline in vitro on neutrophil reactive oxygen production and phagocytic ability assessed by flow cytometry, Clin. Drug Invest., 13(2):99-104, 1997).

Es sind bereits verschiedene PDE 4 Inhibitoren bekannt. Vorrangig handelt es sich dabei um Xanthin-Derivate, Rolipram-Analoge oder Nitraquazon-Abkömmlinge (Übersicht in: Karlsson J-A, Aldos D Phosphodiesterase 4 inhibitors for the treatment of asthma, Exp. Opin. Ther. Patents 1997, 7: 989-1003). Keine dieser Verbindungen konnte bisher bis zur klinischen Anwendung gebracht werden. Es mußte festgestellt werden, daß die bekannten PDE 4 Inhibitoren auch verschiedene Nebenwirkungen wie Nausea und Emesis besitzen, die bisher nicht ausreichend zuruckgedrängt werden konnten. Deshalb ist die Entdeckung neuer PDE 4 Inhibitoren mit besserer therapeutischer Breite erforderlich.

Obwohl Indole bei der Entwicklung neuer Wirkstoffe für verschiedene Indikationen seit vielen Jahren eine wichtige Rolle spielen, sind Hydroxyindole als Inhibitoren der PDE 4 bisher völlig unbekannt.

## Beschreibung der Erfindung

Die Erfindung betrifft substituierte Hydroxyindole der allgemeinen Formel 1,

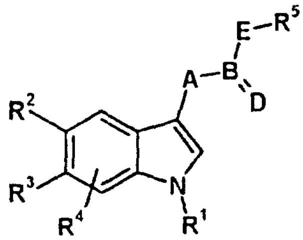

1

worin

**$R^1$** für

$-C_{1...12}$-Alkyl, geradkettig oder verzweigtkettig,
ggf. ein- oder mehrfach substituiert mit -OH, -SH $-NH_2$, $-NHC_{1...6}$-Alkyl, $-N(C_{1...6}$-Alkyl$)_2$, $-NHC_{6...14}$Aryl, $-N(C_{6...14}$Aryl$)_2$, $-N(C_{1...6}$Alkyl$)(C_{6...14}$Aryl), $-NHCOR^6$, $-NO_2$, -CN, -F, -Cl, -Br, -I, $-O-C_{1...6}$-Alkyl, $-O-C_{6...14}$-Aryl, $-O(CO)R^6$, $-S-C_{1...6}$-Alkyl, $-S-C_{6...14}$Aryl, $-SOR^6$, $-SO_3H$, $-SO_2R^6$, $-OSO_2C_{1...6}$Alkyl, $-OSO_2C_{6...14}$Aryl, $-(CS)R^6$, -COOH, $-(CO)R^6$, mono-, bi- oder tricyclische gesättigte oder ein- oder mehrfach ungesättigte Carbocyclen mit 3...14 Ringgliedern, mono-, bi- oder tricyclische gesättigte oder ein- oder mehrfach ungesättigte Heterocyclen mit 5...15 Ringgliedern und 1...6 Heteroatomen, die vorzugsweise N, O und S sind, wobei die $C_{6...14}$Aryl-Gruppen und die eingeschlossenen carbocyclischen und heterocyclischen Substituenten ihrerseits ggf. ein- oder mehrfach mit $R^4$ substituiert sein können,

$-C_{2...12}$-Alkenyl, ein oder mehrfach ungesättigt, geradkettig oder verzweigtkettig,
ggf. ein- oder mehrfach substituiert mit -OH, -SH, $-NH_2$, $-NHC_{1...6}$-Alkyl, $-N(C_{1...6}$-Alkyl$)_2$, $-NHC_{6...14}$Aryl, $-N(C_{6...14}$Aryl$)_2$, $-N(C_{1...6}$Alkyl$)(C_{6...14}$Aryl), $-NHCOR^6$, $-NO_2$, -CN, -F, -Cl, -Br, -I, $-O-C_{1...6}$-Alkyl, $-O-C_{6...14}$-Aryl, $-O(CO)R^6$, $-S-C_{1...6}$-Alkyl, $-S-C_{6...14}$Aryl, $-SOR^6$, $-SO_3H$, $-SO_2R^6$, $-OSO_2C_{1...6}$Alkyl, $-OSO_2C_{6...14}$Aryl, $-(CS)R^6$, -COOH, $-(CO)R^6$, mono-, bi- oder tricyclische gesättigte oder ein- oder mehrfach ungesättigte Carbocyclen mit 3...14 Ringgliedern, mono-, bi- oder tricyclische gesättigte oder ein- oder mehrfach ungesättigte Heterocyclen mit 5...15 Ringgliedern und 1...6 Heteroatomen, die vorzugsweise N, O und S sind, wobei die $C_{6...14}$Aryl-Gruppen und die eingeschlossenen carbocyclischen und heterocyclischen Substituenten ihrerseits ggf. ein- oder mehrfach mit $R^4$ substituiert sein können,

-mono-, bi- oder tricyclische gesättigte oder ein- oder mehrfach ungesättigte Carbocyclen mit 3 ...14 Ringgliedern,
ggf. ein- oder mehrfach substituiert mit -OH, -SH, $-NH_2$, $-NHC_{1...6}$-Alkyl, $-N(C_{1...6}$-Alkyl$)_2$, $-NHC_{6...14}$Aryl, $-N(C_{6...14}$Aryl$)_2$, $-N(C_{1...6}$Alkyl$)(C_{6...14}$Aryl), $-NHCOR^6$, $-NO_2$, -CN, -F, -Cl, -Br -I, $-O-C_{1...6}$-Alkyl, $-O-C_{6...14}$-Aryl, $-O(CO)R^6$, $-S-C_{1...6}$-Alkyl, $-S-C_{6...14}$Aryl, $-SOR^6$, $-SO_3H$, $-SO_2R^6$, $-OSO_2C_{1...6}$Alkyl, $-OSO_2C_{6...14}$Aryl, $-(CS)R^6$, -COOH, $-(CO)R^6$, mono-, bi- oder tricyclische gesättigte oder ein- oder mehrfach ungesättigte Carbocyclen mit 3...14 Ringgliedern, mono-, bi- oder tricyclische gesättigte oder ein- oder mehrfach ungesättigte Heterocyclen mit 5...15 Ringgliedern und 1...6 Heteroatomen, die vorzugsweise N, O und S sind, wobei die $C_{6...14}$Aryl-Gruppen und die eingeschlossenen carbocyclischen und heterocyclischen Substituenten ihrerseits ggf. ein- oder mehrfach mit $R^4$ substituiert sein können,

-mono-, bi- oder tricyclische gesättigte oder ein- oder mehrfach ungesättigte Heterocyclen mit 5...15 Ringgliedern und 1...6 Heteroatomen, die vorzugsweise N, O und S sind,
ggf. ein- oder mehrfach substituiert mit -OH, -SH, $-NH_2$, $-NHC_{1...6}$-Alkyl, $-N(C_{1...6}$-Alkyl$)_2$, $-NHC_{6...14}$Aryl, $-N(C_{6...14}$Aryl$)_2$, $-N(C_{1...6}$Alkyl$)(C_{6...14}$Aryl), $-NHCOR^6$, $-NO_2$, -CN, -F, -Cl, -Br, -I, $-O-C_{1...6}$-Alkyl, $-O-C_{6...14}$-Aryl. $-O(CO)R^6$, $-S-C_{1...6}$-Alkyl, $-S-C_{6...14}$Aryl, $-SOR^6$, $-SO_3H$, $-SO_2R^6$, $-OSO_2C_{1...6}$Alkyl, $-OSO_2C_{6...14}$Aryl, $-(CS)R^6$, -COOH, $-(CO)R^6$, mono-, bi- oder tricyclische gesattigte oder ein- oder mehrfach ungesättigte Carbocyclen mit 3 ..14 Ringgliedern, mono-, bi- oder tricyclische gesattigte oder ein- oder mehrfach ungesättigte Heterocyclen mit 5...15 Ringgliedern und 1...6 Heteroatomen, die vorzugsweise N, O und S sind, wobei die $C_{6...14}$Aryl-Gruppen und die eingeschlossenen carbocyclischen und heterocyclischen Substituenten ihrerseits ggf. ein- oder mehrfach mit $R^4$ substituiert sein können,

-carbo- oder heterocyclische gesättigte oder ein- oder mehrfach ungesättigte Spirocyclen mit 3...10 Ringgliedern, wobei heterocyclische Systeme 1...6 Heteroatome enthalten, die vorzugsweise N, O und S sind,

ggf. ein- oder mehrfach substituiert mit -OH, -SH, $-NH_2$, $-NHC_{1...6}$-Alkyl, $-N(C_{1...6}\text{-Alkyl})_2$, $-NHC_{6...14}$Aryl, $-N(C_{6...14}\text{Aryl})_2$, $-N(C_{1...6}\text{Alkyl})(C_{6...14}\text{Aryl})$, $-NHCOR^6$, $-NO_2$, -CN, -F, -Cl, -Br, -I, $-O-C_{1...6}$-Alkyl, $-O-C_{6...14}$-Aryl, $-O(CO)R^6$, $-S-C_{1...6}$-Alkyl, $-S-C_{6...14}$Aryl, $-SOR^6$, $-SO_3H$, $-SO_2R^6$, $-OSO_2C_{1...6}$Alkyl, $-OSO_2C_{6...14}$Aryl, $-(CS)R^6$, -COOH, $-(CO)R^6$, mono-, bi- oder tricyclische gesättigte oder ein- oder mehrfach ungesättigte Carbocyclen mit 3...14 Ringgliedern, mono-, bi- oder tricyclische gesättigte oder ein- oder mehrfach ungesättigte Heterocyclen mit 5...15 Ringgliedern und 1...6 Heteroatomen, die vorzugsweise N, O und S sind, wobei die $C_{6...14}$Aryl-Gruppen und die eingeschlossenen carbocyclischen und heterocyclischen Substituenten ihrerseits ggf. ein- oder mehrfach mit $R^4$ substituiert sein können,

steht ;

**$R^2$, $R^3$** können Wasserstoff oder -OH sein, wobei mindestens einer von beiden Substituenten -OH sein muß;

**$R^4$** steht für -H, -OH, -SH, $-NH_2$, $-NHC_{1...6}$-Alkyl, $-N(C_{1...6}\text{-Alkyl})_2$, $-NHC_{6...14}$Aryl, $-N(C_{6...14}\text{Aryl})_2$, $-N(C_{1...6}\text{Alkyl})(C_{6...14}\text{Aryl})$, $-NHCOR^6$, $-NO_2$, -CN, -COOH, $-(CO)R^6$, $-(CS)R^6$, -F, -Cl, -Br, -I, $-O-C_{1...6}$-Alkyl, $-O-C_{6...14}$-Aryl, $-O(CO)R^6$, $-S-C_{1...6}$-Alkyl, $-S-C_{6...14}$Aryl, $-SOR^6$, $-SO_2R^6$.

**$R_5$** steht für

-mono-, bi- oder tricyclische gesättigte oder ein- oder mehrfach ungesättigte Carbocyclen mit 3 ...14 Ringgliedern,
ein- oder mehrfach substituiert mit -F, -Cl, -Br, -I,
ggf. ein- oder mehrfach substituiert mit -OH, -SH, $-NH_2$, $-NHC_{1...6}$-Alkyl, $-N(C_{1...6}\text{-Alkyl})_2$, $-NHC_{6...14}$-Aryl, $-N(C_{6...14}\text{-Aryl})_2$,$-N(C_{1...6}\text{-Alkyl})(C_{6...14}\text{-Aryl})$, $-NHCOR^6$, $-NO_2$, -CN, $-O-C_{1...6}$Alkyl, $-O-C_{6...14}$-Aryl, $-O(CO)R^6$, $-S-C_{1...6}$-Alkyl, $-S-C_{6...14}$Aryl, $-SOR^6$, $-SO_3H$, $-SO_2R^6$, $-OSO_2C_{1...6}$-Alkyl, $-OSO_2C_{6...14}$-Aryl, $-(CS)R^6$, -COOH, $-(CO)R^6$, mono-, bi- oder tricyclische gesättigte oder ein- oder mehrfach ungesättigte Carbocyclen mit 3 ...14 Ringgliedern, mono-, bi- oder tricyclische gesättigte oder ein- oder mehrfach ungesättigte Heterocyclen mit 5...15 Ringgliedem und 1...6 Heteroatomen, die vorzugsweise N, O und S sind, wobei die $C_{6...14}$-Aryl-Gruppen und die eingeschlossenen carbocyclischen und heterocyclischen Substituenten ihrerseits ggf. ein- oder mehrfach mit $R^4$ substituiert sein können,

-mono-, bi- oder tricyclische gesättigte oder ein- oder mehrfach ungesättigte Heterocyclen mit 5...15 Ringgliedern und 1 ... 6 Heteroatomen, die vorzugsweise N, O und S sind,
ein- oder mehrfach substituiert mit -F, -Cl, -Br, -I,
ggf. ein- oder mehrfach substituiert mit -OH, -SH, $-NH_2$, $-NHC_{1...6}$-Alkyl, $-N(C_{1...6}\text{-Alkyl})_2$, $-NHC_{6...14}$-Aryl, $-N(C_{6...14}\text{-Aryl})_2$, $-N(C_{1...6}\text{-Alkyl})(C_{6...14}\text{-Aryl})$, $-NHCOR^6$, $-NO_2$, -CN, $-O-C_{1...6}$-Alkyl, $-O-C_{6...14}$-Aryl, $-O(CO)R^6$, $-S-C_{1...6}$-Alkyl, $-S-C_{6...14}$-Aryl, $-SOR^6$, $-SO_3H$, $-SO_2R^6$, $-OSO_2C_{1...6}$-Alkyl, $-OSO_2C_{6...14}$-Aryl, $-(CS)R^6$, -COOH, $-(CO)R^6$, mono-, bi- oder tricyclische gesättigte oder ein- oder mehrfach ungesättigte Carbocyclen mit 3 ...14 Ringgliedem, mono-, bi- oder tricyclische gesättigte oder ein- oder mehrfach ungesättigte Heterocyclen mit 5...15 Ringgliedern und 1...6 Heteroatomen, die vorzugsweise N, O und S sind, wobei die $C_{6...14}$-Aryl-Gruppen und die eingeschlossenen carbocyclischen und heterocyclischen Substituenten ihrerseits ggf. ein- oder mehrfach mit $R^4$ substituiert sein können,

steht,

**$R^6$** kann

-H, $-NH_2$, $-NHC_{1...6}$-Alkyl, $-N(C_{1...6}\text{-Alky})_2$, $-NHC_{6...14}$Aryl, $-N(C_{6...14}\text{Aryl})_2$, $-N(C_{1...6}\text{Alkyl})(C_{6...14}\text{Aryl})$, $-O-C_{1...6}$-Alkyl, $-O-C_{6...14}$-Aryl, $-S-C_{1...6}$-Alkyl, $-S-C_{6...14}$Aryl, $-C_{1...12}$-Alkyl, geradkettig oder verzweigtkettig,
$-C_{2...12}$-Alkenyl, ein oder mehrfach ungesättigt, geradkettig oder verzweigtkettig,
-mono-, bi- oder tricyclische gesättigte oder ein- oder mehrfach ungesättigte
Carbocyclen mit 3 ...14 Ringgliedern,
-mono-, bi- oder tricyclische gesättigte oder ein- oder mehrfach ungesättigte Heterocyclen mit 5...15 Ringgliedern und 1...6 Heteroatomen, die vorzugsweise N, O und S sind,

bedeuten.

**A** steht entweder für eine Bindung, oder für

$-(CH_2)_m-$, $-(CH_2)_m-(CH=CH)_n-(CH_2)_p-$, $-(CHOZ)_m-$, -(C=O)-, -(C=S)-, -(C=N-Z)-,-O-, -S-, -NZ-,

wobei m, p = 0 .. 3 und n = 0... 2 sind und

**Z** für

-H, oder
$-C_{1...12}$-Alkyl, geradkettig oder verzweigtkettig,
$-C_{2...12}$-Alkenyl, ein oder mehrfach ungesättigt geradkettig oder verzweigtkettig,
-mono-, bi- oder tricyclische gesättigte oder ein- oder mehrfach ungesättigte Carbocyclen mit 3 ...14 Ringgliedern,
-mono-, bi- oder tricyclische gesättigte oder ein- oder mehrfach ungesättigte Heterocyclen mit 5...15 Ringgliedern und 1 ..6 Heteroatomen, die vorzugsweise N, O und S sind,

steht.

**B** kann entweder Kohlenstoff oder Schwefel sein, oder -(S=O)- bedeuten.

**D** kann Sauerstoff, Schwefel, $CH_2$ oder N-Z sein,
wobei **D** nur dann S oder $CH_2$ sein kann, wenn **B** Kohlenstoff bedeutet.

**E** kann für eine Bindung stehen, oder aber für
$-(CH_2)_m-$, -O-, -S-, -(N-Z)-, wobei m und **Z** die bereits zuvor beschriebene Bedeutung besitzen.

Weiterhin betrifft die Erfindung die physiologisch vertraglichen Salze der Verbindungen gemäß Formel 1
Die physiologisch verträglichen Salze werden in üblicher Weise durch Neutralisation der Basen mit anorganischen oder organischen Säuren bzw. durch Neutralisation der Säuren mit anorganischen oder organischen Basen erhalten. Als anorganische Säuren kommen zum Beispiel Salzsäure, Schwefelsäure, Phosphorsäure oder Bromwasserstoffsäure, als organische Sauren zum Beispiel Carbon-, Sulfo- oder Sulfonsäuren wie Essigsäure, Weinsaure, Milchsäure, Propionsäure, Glykolsäure, Malonsäure, Maleinsäure, Fumarsäure, Gerbsaure, Succinsäure, Alginsaure, Benzoesaure, 2-Phenoxybenzoesäure, 2-Acetoxybenzosäure, Zimtsäure, Mandelsäure, Zitronensäure, Apfelsaure, Salicylsäure, 3-Aminosalicylsäure, Ascorbinsäure, Embonsaure, Nicotinsaure, Isonicotinsäure. Oxalsaure, Aminosäuren, Methansulfonsäure, Ethansulfonsaure, 2-Hydroxyethansulfonsaure, Ethan-1,2-disulfonsäure, Benzolsulfonsäure, 4-Methylbenzolsulfonsäure oder Naphthalin-2-sulfonsäure in Frage Als anorganische Basen kommen zum Beispiel Natronlauge, Kalilauge, Ammoniak sowie als organische Basen Amine, bevorzugt jedoch tertiäre Amine, wie Trimethylamin, Triethylamin, Pyridin, N,N-Dimethylanilin Chinolin, Isochinolin, α-Picolin, β-Picolin, γ-Picolin, Chinaldin oder Pyrimidin in Frage.
Desweiteren können physiologisch verträgliche Salze der Verbindungen gemäß Formel 1 dadurch gewonnen werden, daß Derivate, die tertiäre Amino-Gruppen besitzen, in an sich bekannter Weise mit Quaternierungsmitteln in die entsprechenden quaternären Ammoniumsalze überführt werden. Als Quaternierungsmittel kommen beispielsweise Alkylhalogenide wie Methyliodid, Ethylbromid und n-Propylchlorid, aber auch Arylalkylhalogenide wie Benzylchlorid oder 2-Phenylethylbromid in Frage.
Weiterhin betrifft die Erfindung von den Verbindungen der Formel 1, die ein asymmetrisches Kohlenstoffatom enthalten, die D-Form, die L-Form und D,L-Mischungen sowie im Falle mehrerer asymmetrischer Kohlenstoffatome die diastereomeren Formen. Diejenigen Verbindungen der Formel 1, die asymmetrische Kohlenstoffatome enthalten und in der Regel als Razemate anfallen, können in an sich bekannter Weise beispielsweise mit einer optisch aktiven Saure in die optisch aktiven Isomeren getrennt werden. Es ist aber auch möglich, von vornherein eine optisch aktive Ausgangssubstanz einzusetzen, wobei dann als Endprodukt eine entsprechende optisch aktive beziehungsweise diastereomere Verbindung erhalten wird .
Für die erfindungsgemäßen Verbindungen wurden pharmakologisch bedeutende Eigenschaften gefunden, die therapeutisch genutzt werden können.
Die erfindungsgemäßen Verbindungen sind Inhibitoren der Freisetzung von TNFα.
Es ist daher Gegenstand dieser Erfindung, daß die Verbindungen gemäß Formel 1 und deren Salze sowie pharmazeutische Zubereitungen, die diese Verbindungen oder deren Salze enthalten, zur Behandlung von Erkrankungen verwendet werden können, bei denen eine Inhibition von TNFα nützlich ist.
Zu diesen Erkrankungen gehoren beispielsweise Gelenksentzundungen einschließlich Arthritis und rheumatoide Arthritis sowie andere arthritische Erkrankungen wie rheumatoide Spondylitis und Osteoarthritis. Weitere Anwendungsmöglichkeiten sind die Behandlung von Patienten, die unter Sepsis, septischem Schock, gramnegativer Sepsis, toxischem Schocksyndrom, Atemnotsyndrom, Asthma oder anderen chronischen pulmonalen Erkrankungen, Knochenresorptions-Krankheiten oder Transplantat-Abstoßungsreaktionen oder anderen Autoimmunerkrankungen, wie Lupus erythematosus, Multiple Sclerose, Glomerulonephritis und Uveitis, Insulin abhängigem Diabetes mellitus sowie chronischer Demyelinisierung leiden.
Außerdem können die erfindungsgemäßen Verbindungen auch zur Therapie von Infektionen wie Virusinfektionen und Parasiten-Infektionen, beispielsweise zur Therapie von Malaria, infektionsbedingtem Fieber, infektionsbedingten Muskelschmerzen, AIDS und Kachexien eingesetzt werden.

Die erfindungsgemäßen Verbindungen sind Inhibitoren der Phosphodiesterase 4.
Es ist daher Gegenstand dieser Erfindung, daß die Verbindungen gemäß Formel 1 und deren Salze sowie pharmazeutische Zubereitungen, die diese Verbindungen oder deren Salze enthalten, zur Behandlung von Erkrankungen verwendet werden können, bei denen eine Inhibition der Phosphodiesterase 4 nützlich ist.
So können die erfindungsgemäßen Verbindungen als Bronchodilatatoren und zur Asthma - Prophylaxe eingesetzt werden.

Die Verbindungen gemäß Formel 1 sind weiterhin Inhibitoren der Akkumulation von Eosinophilen sowie deren Aktivität. Demzufolge können die erfindungsgemäßen Verbindungen auch bei Erkrankungen eingesetzt werden, bei denen Eosinophile eine Rolle spielen. Zu diesen Erkrankungen gehoren beispielsweise entzündliche Atemwegserkrankungen wie Asthma bronchiale, allergische Rhinitis, allergische Konjunktivitis, atopische Dermatitis, Ekzeme, allergische Angiitis, durch Eosinophile vermittelte Entzündungen wie eosinophile Fasciitis, eosinophile Pneumonie und PIE-Syndrom (Pulmonale Infiltration mit Eosinophilie), Urtikaria, ulcerative Colitis, die Crohn-Krankheit und proliferative Hauterkrankungen wie Psoriasis oder Keratosis.

Gegenstand dieser Erfindung ist es, daß die Verbindungen gemäß Formel 1 und deren Salze sowohl die Lipopolysacharid (LPS)-induzierte Freisetzung von TNF$\alpha$ in humanem Blut *in vitro,* als auch die LPS-induzierte pulmonale Neutrophilen-Infiltration bei Frettchen und Hausschweinen *in vivo* inhibieren können. Die Gesamtheit dieser gefundenen pharmakologisch bedeutsamen Eigenschaften belegt, daß die Verbindungen gemäß Formel 1 und deren Salze sowie pharmazeutische Zubereitungen, die diese Verbindungen oder deren Salze enthalten, zur Behandlung von chronisch obstruktiven Lungenerkrankungen therapeutisch genutzt werden können.

Die erfindungsgemäßen Verbindungen besitzen weiterhin neuroprotektive Eigenschaften und können zur Therapie von Krankheiten verwendet werden, bei denen Neuroprotektion nützlich ist. Solche Erkrankungen sind beispielsweise senile Demenz (Alzheimer's Krankheit), Gedächtnisschwund, Parkinson's Krankheit, Depressionen, Schlaganfälle und Claudikatio intermittens.

Weitere Anwendungsmöglichkeiten der erfindungsgemäßen Verbindungen sind die Prophylaxe und Therapie von Prostata-Krankheiten, wie beispielsweise benigne Prostata-Hyperplasie, Pollakisurie, Nocturie sowie zur Behandlung von Blasenschwäche und von durch Harnsteine ausgelösten Koliken.
Schließlich können die erfindungsgemäßen Verbindungen ebenfalls zur Inhibition der Entstehung einer Arzneimittelabhängigkeit bei wiederholtem Einsatz von Analgetika, wie beispielsweise Morphin sowie zur Verringerung der Toleranzentwicklung beim wiederholten Einsatz von diesen Analgetika verwendet werden.

Zur Herstellung der Arzneimittel wird neben den üblichen Hilfsmitteln, Träger- und Zusatzstoffen eine wirksame Dosis der erfindungsgemäßen Verbindungen oder deren Salze verwendet.
Die Dosierung der Wirkstoffe kann je nach Verabfolgungsweg, Alter, Gewicht des Patienten, Art und Schwere der zu behandelnden Erkrankungen und ähnlichen Faktoren variieren.
Die tägliche Dosis kann als einmal zu verabreichende Einzeldosis oder unterteilt in 2 oder mehrere Tagesdosen gegeben werden und beträgt in der Regel 0,001-100 mg .

Als Applikationsform kommen orale, parenterale, intravenöse, transdermale, topische, inhalative und intranasale Zubereitungen in Frage.

Zur Anwendung kommen die üblichen galenischen Zubereitungsformen wie Tabletten, Dragees, Kapseln, dispergierbare Pulver, Granulate, wäßrige Lösungen, wäßrige oder ölige Suspensionen, Sirup, Säfte oder Tropfen.

Feste Arzneiformen können inerte Inhalts- und Trägerstoffe enthalten, wie z. B. Calciumcarbonat, Calciumphosphat, Natriumphosphat, Lactose, Stärke, Mannit, Alginate, Gelatine, Guar-Gummi, Magnesium- oder Aluminiumstearat, Methylcellulose, Talkum, hochdisperse Kieselsäuren, Silikonöl, höhermolekulare Fettsäuren (wie Stearinsäure), Gelatine, Agar-Agar oder pflanzliche oder tierische Fette und Ole, feste hochmolekulare Polymere (wie Polyethylenglykol); für orale Applikation geeignete Zubereitungen können gewünschtenfalls zusätzliche Geschmacks- und/oder Süßstoffe enthalten.

Flüssige Arzneiformen können sterilisiert sein und/oder gegebenenfalls Hilfsstoffe wie Konservierungsmittel, Stabilisatoren, Netzmittel, Penetrationsmittel, Emulgatoren, Spreitmittel, Lösungsvermittler, Salze, Zucker oder Zukkeralkohole zur Regelung des osmotischen Drucks oder zur Pufferung und/oder Viskositätsregulatoren enthalten.
Derartige Zusätze sind zum Beispiel Tartrat- und Citrat-Puffer, Ethanol, Komplexbildner (wie Ethylendiamin-tetraessigsäure und deren nicht-toxische Salze). Zur Regelung der Viskosität kommen hochmolekulare Polymere in Frage wie beispielsweise flüssiges Polyethylenoxid, mikrokristalline Cellulosen Carboxymethylcellulosen, Polyvinylpyrrolidone, Dextrane oder Gelatine Feste Trägerstoffe sind zum Beispiel Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere wie Polyethylenglykol.

Ölige Suspensionen für parenterale oder topische Anwendungen können vegetabile synthetische oder semisynthetische Öle wie beispielsweise flüssige Fettsäureester mit jeweils 8 bis 22 C-Atomen in den Fettsäureketten, zum Beispiel Palmitin-, Laurin-, Tridecyl-, Margarin-, Stearin-, Arachin-, Myristin-, Behen-, Pentadecyl-, Linol-, Elaidin-, Brassidin-, Eruca- oder Olsaure, die mit ein- bis dreiwertigen Alkoholen mit 1 bis 6 C-Atomen wie beispielsweise Methanol, Ethanol, Propanol, Butanol, Pentanol oder deren Isomere, Glycol oder Glycerol verestert sind, sein. Derartige Fettsäureester sind beispielsweise handelsübliche Miglyole, Isopropylmyristat, Isopropylpalmitat, Isopropylstearat, PEG 6-Caprinsäure, Capryl/Caprinsäureester von gesättigten Fettalkoholen, Polyoxyethylenglyceroltrioleate, Ethyloleat, wachsartige Fettsäureester wie künstliches Entenbürzeldrüsenfett, Kokosfettsäure-isopropylester, Ölsäureoleylester, Ölsäuredecylester, Milchsäureethylester, Dibutylphthalat, Adipinsäurediisopropylester, Polyol-Fettsäureester u. a. Ebenso geeignet sind Silikonöle verschiedener Viskositat oder Fettalkohole wie Isotridecylalkohol, 2-Octyldodecanol, Cetylstearyl-Alkohol oder Oleylalkohol, Fettsauren wie beispielsweise Ölsäure. Weiterhin können vegetabile Öle wie Rizinusöl, Mandelöl, Olivenöl, Sesamöl, Baumwollsaatöl, Erdnußöl oder Sojabohnenöl Verwendung finden.

Als Lösungsmittel, Gelbildner und Lösungsvermittler kommen in Frage Wasser oder mit Wasser mischbare Lösungsmittel Geeignet sind zum Beispiel Alkohole wie beispielsweise Ethanol oder Isopropylalkohol, Benzylalkohol, 2-Octyldodecanol, Polyethylenglykole, Phthalate, Adipate, Propylenglykol, Glycerin, Di- oder Tripropylenglykol, Wachse, Methylcellosolve, Cellosolve, Ester, Morpholine, Dioxan, Dimethylsulfoxid, Dimethylformamid, Tetrahydrofuran, Cyclohexanon etc.

Als Filmbildner können Celluloseether verwendet werden, die sich sowohl in Wasser als auch in organischen Lösungsmitteln losen bzw anquellen können, wie beispielsweise Hydroxypropylmethylcellulose, Methylcellulose, Ethylcellulose oder lösliche Stärken.

Mischformen zwischen Gel- und Filmbildnern sind durchaus ebenfalls möglich Hier kommen vor allem ionische Makromolekule zur Anwendung, wie z. B. Natriumcarboxymethylcellulose, Polyacrylsäure, Polymethacrylsaure und deren Salze, Natriumamylopektinsemiglykolat, Alginsäure oder Propylenglykol-Alginat als Natriumsalz, Gummi arabicum, Xanthan-Gummi, Guar-Gummi oder Carrageenan.

Als weitere Formulierungshilfsmittel können eingesetzt werden: Glycerin, Paraffin unterschiedlicher Viskosität, Triethanolamin, Collagen, Allantoin, Novantisolsäure. Auch die Verwendung von Tensiden, Emulgatoren oder Netzmitteln kann zur Formulierung notwendig sein, wie z. B. von Na-Laurylsulfat, Fettalkoholethersulfaten, Di-Na-N-lauryl-β-iminodipropionat, polyoxyethyliertes Rizinusöl oder Sorbitan-Monooleat, Sorbitan-Monostearat, Polysorbaten (z. B. Tween), Cetylalkohol, Lecithin, Glycerinmonostearat, Polyoxyethylenstearat, Alkylphenolpolyglykolether, Cetyltrimethylammoniumchlorid oder Mono/Dialkylpolyglykolether-orthophosphorsäure-monoethanolaminsalzen.
Stabilisatoren wie Montmorillonite oder kolloidale Kieselsäuren zur Stabilisierung von Emulsionen oder zur Verhinderung des Abbaus der aktiven Substanzen wie Antioxidantien, beispielsweise Tocopherole oder Butylhydroxyanisol, oder Konservierungsmittel, wie p-Hydroxybenzoesäureester, können ebenfalls zur Zubereitung der gewünschten Formulierungen gegebenenfalls erforderlich sein.

Zubereitungen zur parenteralen Applikation können in separaten Dosiseinheitsformen wie z. B. Ampullen oder Vials vorliegen. Vorzugsweise werden Lösungen des Wirkstoffes verwendet, bevorzugt wässrige Lösungen und vor allem isotonische Lösungen aber auch Suspensionen. Diese Injektionsformen können als Fertigpräparat zur Verfügung gestellt werden oder erst direkt vor der Anwendung durch Mischen der wirksamen Verbindung, zum Beispiel des Lyophilisats, gegebenenfalls mit weiteren festen Trägerstoffen, mit dem gewünschten Lösungs- oder Suspensionsmittel zubereitet werden.

Intranasale Zubereitungen können als wäßrige oder ölige Losungen bzw als wäßrige oder olige Suspensionen vorliegen Sie können auch als Lyophilisate vorliegen, die vor der Anwendung mit dem geeigneten Lösungs- oder Suspensionsmittel zubereitet werden.

Die Herstellung, Abfullung und Verschließung der Präparate erfolgt unter den üblichen antimikrobiellen und aspetischen Bedingungen.

Die Erfindung betrifft weiterhin Verfahren zur Herstellung der erfindungsgemäßen Verbindungen.
Erfindungsgemäß werden die Verbindungen der allgemeinen Formel <u>1</u>, mit den zuvor dargestellten Bedeutungen von $R^1$, $R^2$, $R^3$, $R^4$, $R^5$, A, B, D und E hergestellt,

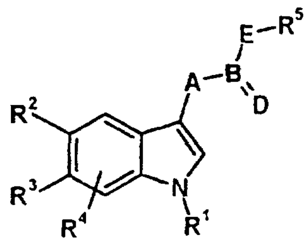

1

indem Verbindungen gemäß Formel 1, für die $R^2$ oder $R^3$ bzw. $R^2$ und $R^3$= -O-$R^7$ bedeuten, durch Abspaltung von **$R^7$** in die erfindungsgemäßen Verbindungen überführt werden.

**$R^7$** steht dabei für als Abgangsgruppe geeignete Substituenten, wie beispielsweise Alkyl-, Cycloalkyl-, Arylalkyl-, Aryl-, Heteroaryl-, Acyl-, Alkoxycarbonyl-, Aryloxycarbonyl-, Aminocarbonyl-, N-substituierte Aminocarbonyl-, Silyl-, Sulfonyl-Gruppen sowie Komplexbildner, wie zum Beispiel Verbindungen der Borsäure, der Phosphorsäure sowie kovalent oder koordinativ gebundene Metalle, wie Zink, Aluminium oder Kupfer.

Eine im Sinne der erfindungsgemäßen Herstellungsverfahren besonders bevorzugte Reaktion zur Abspaltung von **$R^7$** sind Verseifungen mit geeigneten Basen, wie beispielsweise Natronlauge, Kalilauge oder Natriumcarbonat bzw Kaliumcarbonat.

Diese Verseifungen werden für **$R^7$** = Acyl-, Alkoxycarbonyl-, Aryloxycarbonyl-, Aminocarbonyl-, N-substituierte Aminocarbonyl-, Silyl-, Sulfonyl-Gruppen sowie Komplexbildner, wie zum Beispiel Verbindungen der Borsaure, der Phosphorsaure sowie koordinativ gebundene Metalle, wie Zink, Aluminium oder Kupfer bevorzugt verwendet.

Eine im Sinne der erfindungsgemäßen Herstellungsverfahren besonders bevorzugte Reaktion zur Abspaltung von **$R^7$** aus den Verbindungen, in denen **$R^7$** eine Alkyl-, Cycloalkyl-, Arylalkyl-, Aryl-, Heteroaryl-Gruppe ist, sind Etherspaltungen beispielsweise mittels Bromwasserstoffsäure, Chlorwasserstoffsaure, Jodwasserstoffsäure sowie mit aktivierenden Lewis-Sauren, wie beispielsweise $AlCl_3$, $BF_3$, $BBr_3$ oder LiCl, jeweils in Abwesenheit oder in Gegenwart zusätzlicher Aktivatoren, wie beispielsweise Ethan-1,2-dithiol oder Benzylmercaptan sowie Etherspaltungen mittels Wasserstoff, unter erhöhtem Druck oder unter Normaldruck, in Gegenwart eines geeigneten Katalysators, wie beispielsweise Palladium- oder Iridium-Katalysatoren.

Erfindungsgemäß werden die Verbindungen der allgemeinen Formel 1, mit den zuvor dargestellten Bedeutungen von $R^1$, $R^2$, $R^3$, $R^4$, $R^5$, A, B, D und E auch hergestellt, indem durch Umwandlungen der Teilstruktur:

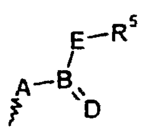

durch an sich bekannte Reaktionen erfindungsgemäße Verbindungen der Formel 1 in andere erfindungsgemäße Verbindungen der Formel 1 uberfuhrt werden.

Besonders bevorzugte Umwandlungsreaktionen mit erfindungsgemäßen Verbindungen der Formel 1 sind beispielsweise für A = -(C=O)- Reduktionen zu

**A** = -(CH-OH)- oder **A** = -$CH_2$- mittels an sich bekannter Reduktionsmittel, wie beispielsweise Natriumborhydrid bzw. durch Hydnerungen, die ggf auch stereoselektiv durchgeführt werden können.

Weitere bevorzugte Umwandlungsreaktionen sind die Überführung von Verbindungen, für die **D** und **E** Sauerstoff bedeutet in Substanzen, bei denen nur noch **D** Sauerstoff bedeutet, **E** aber für -(N-Z)-Steht, wobei **Z** die bereits erklärte Bedeutung hat.

Ausführungsbeispiele

Exemplarische Herstellungsverfahren für erfindungsgemäße Verbindungen der Formel 1 aus Ausgangsstoffen der beschriebenen Art, bei denen $R^7$ eine Alkyl-, Cycloalkyl-, Arylalkyl-, Aryl-, Heteroaryl-Gruppe ist:

Beispiel 1:

**N-(3,5-Dichlorpyridin-4-yl)-2-[1-(4-fluorbenzyl)-5-hydroxy-indol-3-yl]-2-oxo-acetamid (1)**

1,4 g N-(3,5-Dichlorpyridin-4-yl)-2-[1-(4-fluorbenzyl)-5-Methoxy-indol-3-yl)-2-oxo-acetamid (3 mmol) werden

in 100 ml Dichlormethan gelost Die Lösung wird zum Rückfluß erhitzt und unter Rühren mit einer Lösung von 14 mmol $BBr_3$ in 15 ml Dichlormethan versetzt. Das Reaktionsgemisch wird 3 Stunden am Ruckfluß gekocht. Nach Abkühlung wird die Losung mit 200 ml einer wässrigen Natriumhydrogencarbonat-Lösung bei 20 °C 3 Stunden lang intensiv verruhrt. Dabei kristallisiert das Produkt aus Es wird isoliert, bei 60 °C getrocknet und aus 80 ml Ethanol umkristallisiert.
Ausbeute: 1,1 g (80 % d. Theorie)
Schmelzpunkt: 213-214 °C

Beispiel 2

**N-(3,5-Dichlorpyridin-4-yl)-2-[1-(4-fluorbenzyl)-5-hydroxy-indol-3-yl]-2-oxo-acetamid (1)**

5 g (38 mmol) wasserfreies Aluminiumchlorid werden in 50 ml Ethan-1.2-dithiol vorgelegt. Bei 0 °C wird eine Losung von 4,7 g N-(3,5-Dichlorpyridin-4-yl)-2-[1-(4-fluorbenzyl)-5-methoxy-indol-3-yl]-2-oxo-acetamid (10 mmol) in 50 ml Dichlormethan zugegeben. Das Gemisch wird 4 Stunden bei 0 °C gerührt. Unter Ruhren werden bei 0 - 10 °C 50 ml 10 %ige Salzsaure zugetropft Das kristallisierende Produkt wird isoliert, mit Wasser gewaschen und bei 20 °C getrocknet Durch Umkristallisation aus Ethanol (180 ml) wird ein reines Produkt erhalten.
Ausbeute: 3,1 g (67 % der Theorie)
Schmelzpunkt: 212 - 214 °C

Exemplarisches Herstellungsverfahren für erfindungsgemäße Verbindungen der Formel 1 aus Ausgangsstoffen der beschriebenen Art, bei denen $R^7$ eine Acyl-, Alkoxycarbonyl-, Aryloxycarbonyl-, Aminocarbonyl-, N-substituierte Aminocarbonyl-, Silyl-, Sulfonyl-Gruppe ist:

Beispiel 3:

**N-(3,5-Dichlorpyridin-4-yl)-2-[1-(4-fluorbenzyl)-5-hydroxy-indol-3-yl]-2-oxo-acetamid-Na-Salz (2)**

5 g N-(3,5-Dichlorpyridin-4-yl)-2-[ 5-acetoxy-1-(4-fluorbenzyl)-indol-3-yl]-2-oxo-acetamid (10 mmol) werden in 50 ml verdünnter Natronlauge 1 Stunde bei 40 - 50 °C geruhrt. Die Lösung wird unter Kühlung mit Eis mit Salzsäure (10 %ig) neutralisiert und bis zur Trockene eingeengt. Der Ruckstand wird in 80 ml Aceton gelöst. Unlösliche Bestandteile werden abgetrennt. Die klare Losung wird mit einer Losung von 0,4 g NaOH in 3 ml Wasser versetzt und 2 Stunden bei 20 °C gerührt. Das kristallisierte Produkt wird isoliert. mit Aceton gewaschen und bei 60 °C getrocknet
Ausbeute. 2,44 g (51 % der Theorie)
Schmelzpunkt : 265 °C

Exemplarisches Herstellungsverfahren für erfindungsgemäße Verbindungen der Formel 1 aus anderen erfindungsgemäßen Verbindungen der Formel 1.

Beispiel 4

**N-(3,5-Dichlorpyridin-4-yl)-2-[1-(4-fluorbenzyl)-5-hydroxy-indol-3-yl]-2-hydroxy-acetamid (3)**

1 g N-(3,5-Dichlorpyridin-4-yl)-2-[1-(4-fluorbenzyl)-5-hydroxy-indol-3-yl]-2-oxo-acetamid (1.2 mmol) werden in 75 ml Methanol suspendiert. Nach Zugabe einer Losung von 0,2 g Natriumborhydrid in 3 ml verdünnter Natronlauge wird das Reaktionsgemisch 6 Stunden bei 20 °C gerührt. Nachdem das Lösungsmittel abdestilliert wurde, wird der Rückstand aus 40 ml Ethanol umkristallisiert.
Ausbeute: 0,5 g (50 % der Theorie)
Schmelzpunkt: 205 - 207 °C

Unter Verwendung der angegebenen beispielhaften Varianten können zahlreiche weitere Verbindungen der Formel 1 hergestellt werden, von denen folgende beispielhaft angeführt werden :

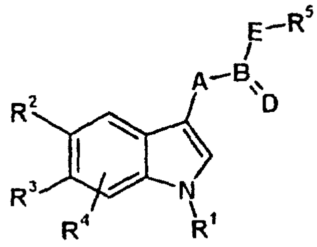

1

| Verb. | $R^1$ | $R^2$ | $R^3$ | $R^4$ | $R^5$ | A | B | D | E | Schmelzpkt. [°C] |
|---|---|---|---|---|---|---|---|---|---|---|
| 1 | 4-Fluor-benzyl- | -OH | -H | -H | 3,5-Dichlor-4-pyridyl- | -(C=O)- | C | O | -(N-H)- | 215 |
| 2 | 4-Fluor-benzyl- | $-O^- Na^+$ | -H | -H | 3,5-Dichlor-4-pyridyl- | -(C=O)- | C | O | -(N-H)- | 265 |
| 3 | 4-Fluor-benzyl- | -OH | -H | -H | 3,5-Dichlor-4-pyridyl- | -(CHOH)- | C | O | -(N-H)- | 205 - 207 |
| 5 | 2,6-Difluor-benzyl- | -OH | -H | -H | 3,5-Dichlor-4-pyridyl- | -(C=O)- | C | O | -(N-H)- | 266 - 268 |
| 6 | 3-Nitro-benzyl- | $-O^- Na^+$ | -H | -H | 3,5-Dichlor-4-pyridyl- | -(C=O)- | C | O | -(N-H)- | 235 - 238 zers. |
| 7 | *n*-Propyl- | -OH | -H | -H | 3,5-Dichlor-4-pyridyl- | -(C=O)- | C | O | -(N-H)- | 280 - 282 |
| 8 | Isopropyl- | -OH | -H | -H | 3,5-Dichlor-4-pyridyl- | -(C=O)- | C | O | -(N-H)- | 245 - 247 |
| 9 | Cyclopentyl-methyl- | -OH | -H | -H | 3,5-Dichlor-4-pyridyl- | -(C=O)- | C | O | -(N-H)- | 246 - 248 |
| 10 | 4-Fluor-benzyl- | -OH | -H | -H | 2,6-Dichlor-phenyl- | -(C=O)- | C | O | -(N-H)- | 216 - 218 |
| 11 | 4-Fluor-benzyl- | -OH | -H | -H | 2,6-Dichlor-4-trifluor-methyl-phenyl- | -(C=O)- | C | O | -(N-H)- | 199 - 201 |
| 12 | 4-Fluor-benzyl- | -OH | -H | -H | 2,6-Dichlor-4-trifluor-methoxy-phenyl- | -(C=O)- | C | O | -(N-H)- | 176 - 178 |
| 13 | 4-Fluor-benzyl- | -H | -OH | -H | 3,5-Dichlor-4-pyridyl- | -(C=O)- | C | O | -(N-H)- | 212 - 213 |
| 14 | 4-Methoxy-benzyl | -OH | -H | -H | 3,5-Dichlor-4-pyridyl- | - | C | O | -(N-H)- | 239 - 241 |

Die erfindungsgemäßen Verbindungen sind starke Inhibitoren der Phosphodiesterase 4 und der TNFα Freisetzung. Ihr therapeutisches Potential wird *in vivo* beispielsweise durch die Hemmung der asthmatischen Spätphase-Reaktion (Eosinophilie) am Meerschweinchen sowie durch die Beeinflussung der Allergen-induzierten vaskulären Permeabilität an aktiv sensibilisierten Brown-Norway Ratten belegt.

Inhibition der Phosphodiesterase

Die PDE 4-Aktivität wird in Enzympraparationen aus humanen polymorphkernigen Lymphocyten (PMNL) bestimmt, die PDE 2, 3 und 5-Aktivität mit PDE aus humanen Thrombocyten. Humanes Blut wurde mit Citrat anticoaguliert. Durch eine Zentrifugation bei 700 x g für 20 Minuten bei RT wird das thrombocytenreiche Plasma im Überstand von den Erythrocyten und Leukocyten getrennt. Die Thrombocyten werden durch Ultraschall lysiert und im PDE 3 und PDE 5-Assay eingesetzt. Für die Bestimmung der PDE 2-Aktivität wird die cytosolische Thrombocytenfraktion über einer Anionenaustauschersäule mittels NaCl-Gradienten gereinigt und der PDE 2-Peak wird für den Assay gewonnen. Die PMNLs für die PDE 4-Bestimmung werden durch eine folgende Dextransedimentation und anschließende Gradienzentrifugation mit Ficoll-Paque isoliert. Nach einem zweimaligen Waschen der Zellen werden die noch enthaltenden Erythrocyten durch die Zugabe von 10 ml hypotonischem Puffer (155 mM NH4Cl, 10 mM NaHCO3, 0,1 mM EDTA, pH=7,4) innerhalb von 6 Minuten bei 4 °C lysiert. Die noch intakten PMNLs werden noch zwei Mal mit PBS gewaschen und mittels Ultraschall lysiert. Der Überstand einer einstündigen Zentrifugation bei 4 °C bei 48000 x g enthalt die cytosolische Fraktion der PDE 4 und wird für die PDE 4-Messungen eingesetzt.

Die Phosphodiesterase-Aktivität wird mit einigen Modifizierungen nach der von Thompson et al. beschriebenen Methode bestimmt (Thompson. W.J. ; Appleman, M.M., Assay of cyclic nucleotide phosphodiesterase and resolution of multiple molecular forms of the enzyme Adv. Cycl. Nucl. Res 1979. 10, 69-92 ).
Die Reaktionsmischungen enthalten 50 mM Tris-HCl (pH 7,4), 5 mM $MgCl_2$, die Inhibitoren in variablen Konzentration, die entsprechende Enzympräparation sowie die zur Erfassung der einzelnen Isoenzyme notwendigen weiteren Komponenten (siehe unten). Durch die Zugabe des Substrates 0,5 μM [$^3$H]-cAMP oder [$^3$H]-cGMP (ca. 6000 CPM/Test) wird die Reaktion gestartet. Das Endvolumen beträgt 100 ml. Testsubstanzen werden als Stammlösungen in DMSO angesetzt. Die DMSO-Konzentration im Reaktionsgemisch ist 1% v/v. Bei dieser DMSO-Konzentration wird die PDE-Aktivität nicht beeinflußt. Nach dem Start der Reaktion mittels Substrat-Zugabe werden die Proben 30 Minuten bei 37°C inkubiert Durch ein Erhitzen der Testtubes für 2 Minuten auf 110 °C wird die Reaktion gestoppt. Die Proben bleiben für weitere 10 Minuten im Eis. Nach der Zugabe von 30 μl 5'-Nukleotidase (1 mg/ml, aus einer Schlangengiftsuspension aus Crotalus adamanteus) erfolgt eine Inkubation für 10 Minuten bei 37°C. Die Proben werden auf Eis abgestoppt, jeweils 400 μl einer Mischung aus Dowex-Wasser-Ethanol (1+1+1) zugegeben, gut gemixt und wieder 15 Minuten auf Eis inkubiert. Die Reaktionsgefäße werden 20 Minuten bei 3000 x g zentrifugiert 200 μl Aliquotes des Überstandes werden direkt in Szintillationgefäße überfuhrt. Nach der Zugabe von 3 ml Szintillator werden die Proben im Betacounter gemessen.

Für die Bestimmung der PDE 4, 3 und 2-Aktivitat wird als Substrat [$^3$H]-cAMP, für die Bestimmung der PDE 5-Aktivität [$^3$H]-cGMP verwendet. Die jeweils unspezifischen Enzymaktivitäten werden in Gegenwart von 100 μM Rolipram bei der PDE 4 und in Gegenwart von 100 μM IBMX bei der Bestimmung der PDE 3 und 5 ermittelt und von den Testwerten subtrahiert. Die inkubationsansätze des PDE 3-Assays enthalten 10 μM Rolipram, um eventuelle Verunreinigungen durch die PDE 4 zu hemmen Die PDE 2 wird mit einem SPA-Assay der Firma Amersham getestet. In Gegenwart des Aktivators der PDE 2 (5 μM cGMP) wird der Assay durchgeführt.

Für die erfindungsgemäßen Verbindungen wurden bezüglich der Inhibition der Phosphodiesterase 4 $IC_{50}$-Werte im Bereich von $10^{-9}$ bis $10^{-5}$ M bestimmt. Die Selektivität gegenüber den PDE - Typen 2, 3 und 5 betragt Faktor 100 bis 10 000.

Hemmung der TNFα Freisetzung aus Zellen nasaler Polypen

Die Versuchsanordnung entspricht Im Wesentlichen der von Campbell, A.M. und Bousquet J (Anti-allergic activity of $H_1$-blockers Int Arch. Allergy Immunol., 1993, 101, 308-310) beschriebenen Methode. Das Ausgangsmaterial bilden nasale Polypen (OP-Material) von Patienten die sich einer chirurgischen Behandlung unterzogen haben.
Das Gewebe wird mit RPMI 1640 gewaschen und anschließend mit Protease (2.0 mg/ml, Collagenase (1.5 mg/ml), Hyaluronidase (0.75 mg/ml) und DNAse (0.05 mg/ml) über 2 h bei 37 °C aufgeschlossen (1 g Gewebe auf 4 ml RPMI 1640 mit Enzymen). Die erhaltenen Zellen, eine Mischung aus Epithelzellen, Monozyten, Makrophagen, Lymphozyten, Fibroblasten und Granulozyten, werden filtriert und durch wiederholtes Zentrifugieren in Nährlösung gewaschen, durch Zugabe von humanem IgE passiv sensibilisiert und die Zellsuspension auf eine Konzentration von 2 Mio Zellen/ml in RPMI 1640 (erganzt mit Antibiotika, 10 % fetalem Kälberserum, 2 mM Glutamin und 25 mM Hepes) eingestellt. Diese Suspension wird auf 6-Well-Zellkulturplatten (1 ml/Well) verteilt. Die Zellen werden mit den Prüfsubstanzen in verschiedenen Endkonzentrationen 30 min vorinkubiert und anschließend durch Zugabe von Anti-IgE (7,2 μg/ml) zur TNFα

Freisetzung angeregt. Die maximale Freisetzung in das Nährmedium erfolgt nach ca 18 Stunden. In diesem Zeitraum werden die Zellen bei 37 °C und 5 % $CO_2$ inkubiert. Das Nährmedium (Überstand) wird durch Zentrifugation gewonnen (5 min 4000 U/min) und bis zur Zytokinbestimmung bei -70 °C gelagert. Die Bestimmung von TNFα im Überstand erfolgt mit sog Sandwich-ELISAs (Grundmaterial Pharmingen), mit denen Konzentrationen des Zytokins im Bereich von 30-1000 pg/ml nachgewiesen werden können.
Nicht mit Anti-IgE stimulierte Zellen produzieren kaum TNFα, stimulierte Zellen dagegen sezernieren große Mengen an TNFα, was z.B. durch PDE4 Inhibitoren dosisabhangig vermindert werden kann. Aus der prozentualen Hemmung (TNFα-Freisetzung der mit Anti-IgE stimulierte Zellen = 100 %) der gepruften Substanzen bei verschiedenen Konzentrationen wird die $IC_{50}$ (concentration at 50 % inhibition) berechnet.

Für die erfindungsgemäßen Verbindungen wurden $IC_{50}$ - Werte im Bereich von $10^{-7}$ bis $10^{-5}$ M bestimmt.

Hemmung der Spätphasen-Eosinophilie 24 h nach inhalativer Ovalbuminchallenge an aktiv sensibilisierten Meerschweinchen

Die Hemmung der pulmonalen Eosinophilen-Infiltration durch die Substanzen wird in einem *in vivo* Test an aktiv gegen Ovalbumin (OVA) sensibilisierten männlichen Dunkin-Hartley Meerschweinchen (200-250 g) geprüft. Die Sensibilisierung erfolgt durch zwei intraperitoneale Injektionen einer Suspension von 20 µg OVA zusammen mit 20 mg Aluminiumhydroxid als Adjuvans in 0,5 ml physiologischer Kochsalzlösung pro Tier an zwei aufeinanderfolgenden Tagen. 14 Tage nach der zweiten Injektion werden die Tiere mit Mepyramin maleat (10 mg/kg i.p.) vorbehandelt, um sie vor dem anaphylaktischen Tod zu schützen. 30 Minuten später werden die Tiere in einer Plastikbox für 30 sec einem OVA-Aerosol ausgesetzt (0,5 mg/ml) das von einem mit Pressluft (19,6 kPa) getriebenen Vernebler erzeugt wird (Allergen-Challenge). Kontrolltiere werden mit physiologischer Kochsalzlösung vernebelt. 24 Stunden nach der Challenge werden die Tiere mit einer Überdosis Ethylurethan (1,5 g/kg Körpergewicht i.p.) narkotisiert und eine bronchoalveolare Lavage (BAL) mit 2 x 5 ml physiologischer Kochsalzlösung durchgeführt. Die BAL-Flüssigkeit wird gesammelt, bei 300 rpm für 10 min zentrifugiert und anschließend das Zellpellet in 1 ml physiologischer Kochsalzlösung resuspendiert. Die Eosinophilen in der BAL werden mit einem automatischen Zelldifferenzierungsgerät (Bayer Diagnostics Technicon H1) gezählt. Bei jedem Test werden 2 Kontrollgruppen (Vernebelung mit physiologischer Kochsalzlösung und Vernebelung mit OVA-Lösung) mitgefuhrt.
Die prozentuale Hemmung der Eosinophilie der mit Substanz behandelten Versuchsgruppe wird nach folgender Formel berechnet:

$$\% \text{ Hemmung} = 100 - \frac{100 \cdot (B - C)}{(A - C)}$$

A = Eosinophile in der Kontrollgruppe mit OVA-Challenge und Vehicel
B = Eosinophile in der mit Substanz behandelten Gruppe mit OVA-Challenge
C = Eosinophile in der Kontrollgruppe mit 0.9 %iger NaCl-Challenge und Vehicel

Die Testsubstanzen werden intraperitoneal oder oral als Suspension in 10 % Polyethylenglycol 300 und 0.5 %iger 5-Hydroxyethylcellulose 2 Stunden vor der Allergen-Challenge appliziert. Die Kontrollgruppen werden entsprechend der Applikationsform der Testsubstanz mit dem Vehicel behandelt.

Die erfindungsgemäßen Verbindungen hemmen die Spätphasen-Eosinophilie nach intraperitonealer Applikation von 10 mg/kg um 30% bis 80% und nach oraler Applikation von 30 mg/kg um 40% bis 70 %.
Die erfindungsgemäßen Verbindungen sind somit besonders geeignet für die Herstellung von Arzneimitteln zur Behandlung von Erkrankungen, die mit dem Wirken von Eosinophilen verbunden sind.

Beeinflussung der Allergen-induzierten vaskulären Permeabilität an aktiv sensibilisierten Brown-Norway Ratten

Männliche Brown-Norway Ratten im Gewicht von 280-300 g werden an 2 aufeinanderfolgenden Tagen durch intraperitoneale Injektion einer Suspension von 1 mg Ovalbumin zusammen mit 100 mg Aluminiumhydroxid in 1 ml/Tier aktiv sensibilisiert. Drei Wochen nach der Sensibilisierung werden die Ratten mit Natriumthiopental narkotisiert und in Rückenlage fixiert. Zur Perfusion der Nasenhöhle wurde in die Trachea ein Polyethylenkatheter retrograd bis zur inneren Öffnung der Choanen vorgeschoben, so daß die Lösung durch die Nasenlöcher austropfen konnte. Ein kurzer Trachealkatheter wurde orthograd in die Trachea eingebunden, um die Atmung zu ermöglichen. Zur Perfusion wurde Phosphat gepufferte Kochsalzlosung (PBS) kontinuierlich mit einer Rollerpumpe durch die Nasenhöhle gepumpt (0,5 ml/min) und durch einen Fraktionssammler gesammelt. Evans Blue wurde als Plasmarnarker verwendet und intravenös (je 1 ml/Tier einer 1%-igen Losung in PBS) durch einen in der Vena jugularis liegenden Katheter injiziert.
Die Substanzapplikation erfolgte topisch. Bei dieser Applikation wurde die Testsubstanz dem Perfusionsmedium (PBS)

zugesetzt. Die nasale Schleimhaut wurde 30 Min lang mit PDE4-Inhibitor-haltiger Losung perfundiert Anschließend wurde Evans blue unmittelbar vor Beginn der Perfusion mit Ovalbumin-haltiger Losung (Challenge) injiziert. Nach Beginn der Ovalbuminchallenge (10 mg/ml Ovalbumin in PBS gelost) wurden aller 15 min Fraktionen in den Fraktionssammler über einen Zeitraum von 60 min gesammelt. Die Evans Blue-Konzentration in den Perfusaten wurde mit dem Photometer Digiscan bei einer Wellenlänge von 620 nm gemessen. Dabei wurden die Blankwerte automatisch abgezogen. Der Wirkungsverlauf über 60 min wurde mit einem AUC-Programm berechnet. Die Substanzwirkung der Präparategruppe wurde gegen Vehikelkontrollen in % berechnet.

Für die erfindungsgemäßen Verbindungen wurden $IC_{50}$ - Werte im Bereich von $10^{-8}$ bis $10^{-5}$ M bestimmt.

Die Verwendbarkeit der erfindungsgemäßen Verbindungen gemaß Formel 1 für die Therapie chronisch obstruktiver Lungenkrankheiten wird durch die Hemmung der LPS-induzierten TNFα Freisetzung in humanem Blut sowie durch die Hemmung der LPS-induzierten pulmonalen Neutrophielen-infiltratron bei Frettchen und Hausschweinen belegt.

Die Stimulation von isolierten Leukozyten zur Cytokinfreisetzung kann auf verschiedenen Wegen erfolgen Ein für die Untersuchung der TNFα-Freisetzung geeigneter Stimulus sind Lipopolysaccharide (LPS). LPS ist Bestandteil der Bakterienzellwände und wird durch Abtotung der Bakterien (Antibiotika oder Immunsystem) freigesetzt. LPS stimuliert besonders die Aktivität der phagozytierenden Leukozyten (Gewebsmakrophagen, Granulozyten, Monozyten) und verursacht die Einwanderung von Leukozyten aus der Blutbahn in das betroffenen Gewebe. Ein für diese Mechanismen wichtiges Cytokin ist das TNFα., welches von den betroffenen Zellen (Hauptquelle sind die Monozyten und Makrophagen) in großen Mengen ausgeschüttet wird und neben anderen Mediatoren die Entzundung initiiert und erhält.

<u>LPS-induzierte TNFα-Freisetzung im 1:5 verdünnten Humanblut</u>

Für die Untersuchung zur Beeinflussung der TNFα-Freisetzung wurde Blut von verschiedenen Spendern gewonnen (Gerinnungshemmung mittels Citrat) und mit Zellkulturmedium RPMI 1640 1:5 verdünnt. Die Testsubstanzen wurden den Proben in verschiedenen Konzentrationen vor der LPS-Challenge zugesetzt. Die Stimulation der Leukozyten erfolgte 30 min spater mit Lipopolysaccharid (LPS) *von Salmonella abortus equi* in einer Endkonzentration von 10 µg/ml. Nach Inkubation der Testansätze über 24 Stunden bei 37°C und 5% $CO_2$ im Brutschrank wurde das verdünnte Blut zentrifugiert und im zellfreien Überstand die TNFα-Konzentration mittels ELISA gemessen.
Für die erfindungsgemäßen Verbindungen wurden $IC_{50}$ -Werte im Bereich von $10^{-7}$ bis $10^{-5}$ M bestimmt. Für die Verbindung gemäß Ausführungsbeispiel 1 wurde beispielsweise ein $IC_{50}$ -Wert von 0,8 µmol/l bestimmt. Im Vergleich dazu wurde mit dem Referenzstandard SB 207499 ein $IC_{50}$ -Wert von 7,0 µmol/l ermittelt.

<u>Hemmung der Lipopolysaccharid (LPS)-induzierten Neutrophilie an Frettchen</u>

Die Hemmung der pulmonalen Neutrophilen-Infiltration durch die Substanzen wird in einem *in vivo* Test an männlichen Frettchen (0,6 - 2 kg) geprüft. Die Versuchstiere werden mit Pentobarbital-Natrium (40 mg/kg Körpergewicht i.p.) narkotisiert, einzeln in eine geschlossene Vernebelungsbox von 5 l Rauminhalt gelegt und für 10 Minuten einem ultraschallvernebelten Aerosol aus 0,01 %iger LPS (Lipopolysaccharid)-Lösung (zusätzlich 0,1% Hydroxylamin in PBS) aussetzt. Das Aerosol wird von einem mit Pressluft (0,2 Mpa) getriebenen Vernebler erzeugt Kontrolltiere werden mit einem Aerosol aus physiologischer Kochsalzlösung behandelt. Während des gesamten Vorganges werden die Tiere beobachtet und nach Frischluftzufuhr aus der Vernebelungsbox entnommen. Vernebeltes LPS löst beim Einatmen sofort eine Entzundung der Atemwege aus, die gekennzeichnet ist durch eine massive Einwanderung von neutrophilen Granulozyten in die Lungen der Versuchstiere. Die Neutrophilie erreicht ihr Maximum 4 bis 6 Stunden nach der LPS-Exposition. Um die Anzahl der eingewanderten neutrophilen Granulozyten messen zu können, werden die Tiere 6 Stunden nach der LPS-Provokation mit einer Uberdosis Ethylurethan (1,5 g/kg Körpergewicht i.p.) narkotisiert und eine bronchioalveoläre Lavage (Spülung der Lunge, BAL) mit 2 x 10 ml physiologischer Kochsalzlösung durchgeführt. Die Anzahl der Zellen in der gepoolten original BAL-Flüssigkeit (100 µl) werden mit dem automatischen Zellzählgerat Technicon H1E (Firma Bayer Diagnostic) bestimmt und die verschiedenen Leukozyten pro µl differenziert.
Bei jedem Test werden 2 Kontrollgruppen (Vernebelung mit physiologischer Kochsalzlosung oder mit LPS-Losung) mitgeführt. Antiinflammatorisch wirkende Substanzen, besonders solche, die die TNFα-Freisetzung oder die Funktion der neutrophilen Granulozyten beeinflussen, hemmen die Einwanderung der Leukozyten. Die Hemmung der Einwanderung wird durch den Vergleich der Anzahl eingewanderter Neutrophiler bei unbehandelten Versuchstieren (mit und ohne LPS-Provokation) ermittelt.

Für die erfindungsgemäßen Verbindungen wurden $ID_{50}$ -Werte im Bereich von 1 bis 20 mg/kg i.p. bestimmt. Die Verbindung gemäß Ausführungsbeispiel 1 wurde beispielsweise in den Dosen 1, 3 und 10 mg/kg i.p. 2 Stunden vor der LPS-Provokation an bis zu 3 Versuchstieren je Dosis appliziert. Die Neutrophilie in der BAL wurde dosisabhängig gehemmt (18%, 64% und 78%). Die $ID_{50}$ beträgt 2,4 mg/kg i.p..

Die Applikation des selektiven PDE 4 inhibitors RPR-73401 (Referenzsubstanz) bewirkte in der Dosis 1 mg/kg i.p eine Hemmung der Neutrophilie von 49%.

Für die intrapulmonale Applikation wird den Tieren in Narkose (40 mg/kg i.p. Pentobarbital-Natrium, 3%ig, 1,3 ml/kg) die Trachea eröffnet, ein 7 cm langer PVC Katheter eingebunden und die Testsubstanzen 2 Stunden vor LPS-Provokation in Pulverform (vermischt mit Lactose ad 20 mg/kg) mittels Spritze intrapulmonal appliziert.

Die intrapulmonale Gabe der Verbindung gemäß Ausführungsbeispiel 1 in den Dosen 1, 3 und 10 mg/kg hemmt die LPS induzierte Neutrophilie dosisabhängig (43%, 65% und 100%) Die $ID_{50}$ beträgt 1,65 mg/kg i pulm..

<u>LPS-induzierte Neutrophilie am Hausschwein</u>

Eine Lungen-Neutrophilie kann beim Hausschwein ähnlich wie beim Frettchen ausgelöst werden Die Tiere werden narkotisiert (Pentobarbital 10 mg/kg i.v.) und intubiert. Mit einem Bronchoskop wird eine partielle bronchoalveoläre Lavage durchgeführt, um den Anteil der neutrophilen Granulozyten unter physiologischen Bedingungen zu erfassen. Anschließend wird die Prüfsubstanz appliziert und über den Trachealtubus atmen die Tiere ultraschallvernebeltes Aerosol aus 0,03%iger LPS (Lipopolysaccharid)-Losung (zusätzlich 0,1% Hydroxylamin in PBS) über 20 min ein. Das inhalierte LPS löst eine reaktive Entzundung der Atemwege aus und es wandern massiv neutrophile Granulozyten ein. Die Neutrophilie erreicht ihr Maximum 4 bis 6 Stunden nach der LPS-Exposition. Nach 6 Stunden wird die bronchioalveoläre Lavage wiederholt und der Anstieg der Neutrophilenzahl rechnerisch ermittelt.

Die Tierart Schwein ist für diese Untersuchungen besonders geeignet, da große anatomische und physiologische Ähnlichkeiten zum Menschen bestehen.

Für die erfindungsgemäßen Verbindungen wurden bei intrapulmonaler Gabe von 10 mg/Tier Hemmungen der LPS-induzierten Neutropilie von 20% bis 65% bestimmt.

Die intrapulmonale Gabe der Verbindung gemäß Ausführungsbeispiel 1 in der Dosierung 10 mg/Tier (ca 0,75 mg/kg) hemmte die LPS-induzierte Lungenneutrophilie mit 51 %.

## Patentansprüche

**1.** Hydroxyindole der Formel <u>1</u>

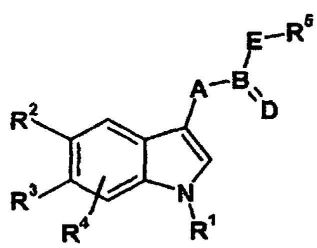

<u>1</u>

worin

**$R^1$** steht für

$-C_{1...12}$-Alkyl, geradkettig oder verzweigtkettig,

ggf. ein- oder mehrfach substituiert mit -OH, -SH, $-NH_2$, $-NHC_{1...6}$-Alkyl, $-N(C_{1...6}\text{-Alkyl})_2$, $-NHC_{6...14}$Aryl, $-N(C_{6...14}\text{Aryl})_2$, $-N(C_{1...6}\text{Alkyl})(C_{6...14}\text{Aryl})$, $-NHCOR^6$, $-NO_2$, -CN, -F, -Cl, -Br, -I, $-O-C_{1...6}$-Alkyl, $-O-C_{6...14}$-Aryl, $-O(CO)R^6$, $-S-C_{1...6}$-Alkyl, $-S-C_{6...14}$Aryl, $-SOR^6$, $-SO_3H$, $-SO_2R^6$, $-OSO_2C_{1...6}$Alkyl, $-OSO_2C_{6...14}$Aryl, $-(CS)R^6$, -COOH, $-(CO)R^6$, mono-, bi- oder tricyclische gesättigte oder ein- oder mehrfach ungesättigte Carbocyclen mit 3 ...14 Ringgliedern, mono-, bi- oder tricyclische gesättigte oder ein- oder mehrfach ungesättigte Heterocyclen mit 5...15 Ringgliedem und 1...6 Heteroatomen, die vorzugsweise N, O und S sind, wobei die $C_{6...14}$Aryl-Gruppen und die eingeschlossenen carbocyclischen und heterocyclischen Substituenten ihrerseits ggf. ein- oder mehrfach mit $R^4$ substituiert sein können,

$-C_{1...12}$-Alkenyl, ein oder mehrfach ungesättigt, geradkettig oder verzweigtkettig, ggf. ein- oder mehrfach substituiert mit -OH, -SH, $-NH_2$, $-NHC_{1...6}$-Alkyl, $-N(C_{1...6}\text{-Alkyl})_2$, $-NHC_{6...14}$Aryl, -N

$(C_{6...14}Aryl)_2$, $-N(C_{1...6}Alkyl)(C_{6...14}Aryl)$, $-NHCOR^6$, $-NO_2$, -CN, -F, -Cl, -Br, -I, $-O-C_{1...6}$-Alkyl, $-O-C_{6...14}$-Aryl, $-O(CO)R^6$, $-S-C_{1...6}$-Alkyl, $-S-C_{6...14}$Aryl, $-SOR^6$, $-SO_3H$, $-SO_2R^6$, $-OSO_2C_{1...6}$Alkyl, $-OSO_2C_{6...14}$Aryl, $-(CS)R^6$, -COOH, $-(CO)R^6$, mono-, bi- oder tricyclische gesättigte oder ein- oder mehrfach ungesättigte Carbocyclen mit 3...14 Ringgliedem, mono-, bi- oder tricyclische gesättigte oder ein- oder mehrfach ungesättigte Heterocyclen mit 5...15 Ringgliedern und 1...6 Heteroatomen, die vorzugsweise N, O und S sind, wobei die $C_{6...14}$Aryl-Gruppen und die eingeschlossenen carbocyclischen und heterocyclischen Substituenten ihrerseits ggf. ein- oder mehrfach mit $R^4$ substituiert sein können,

-mono-, bi- oder tricyclische gesättigte oder ein- oder mehrfach ungesättigte Carbocyclen mit 3 ... 14 Ringgliedern,

ggf. ein- oder mehrfach substituiert mit -OH, -SH, $-NH_2$, $-NHC_{1...6}$-Alkyl, $-N(C_{1...6}\text{-Alkyl})_2$, $-NHC_{6...14}$Aryl, $-N(C_{6...14}Aryl)_2$, $-N(C_{1...6}Alkyl)(C_{6...14}Aryl)$, $-NHCOR^6$, $-NO_2$, -CN, -F, -Cl, -Br, -I, $-O-C_{1...6}$-Alkyl, $-O-C_{6...14}$-Aryl, $-O(CO)R^6$, $-S-C_{1...6}$-Alkyl, $-S-C_{6...14}$Aryl, $-SOR^6$, $-SO_3H$, $-SO_2R^6$, $-OSO_2C_{1...6}$Alkyl, $-OSO_2C_{6....14}$Aryl, $-(CS)R^6$, -COOH, $-(CO)R^6$, mono-, bi- oder tricyclische gesättigte oder ein- oder mehrfach ungesättigte Carbocyclen mit 3...14 Ringgliedern, mono-, bi- oder tricyclische gesättigte oder ein- oder mehrfach ungesättigte Heterocyclen mit 5...15 Ringgliedern und 1...6 Heteroatomen, die vorzugsweise N, O und S sind, wobei die $C_{6...14}$Aryl-Gruppen und die eingeschlossenen carbocyclischen und heterocyclischen Substituenten ihrerseits ggf. ein- oder mehrfach mit $R^4$ substituiert sein können,

-mono-, bi- oder tricyclische gesättigte oder ein- oder mehrfach ungesättigte Heterocyclen mit 5... 15 Ringgliedem und 1...6 Heteroatomen, die vorzugsweise N, O und S sind,

ggf. ein- oder mehrfach substituiert mit -OH, -SH, $-NH_2$, $-NHC_{1...6}$-Alkyl, $-N(C_{1...6}\text{-Alkyl})_2$, $-NHC_{6...14}$Aryl, $-N(C_{6...14}Aryl)_2$, $-N(C_{1...6}Alkyl)(C_{6...14}Aryl)$, $-NHCOR^6$, $-NO_2$, -CN, -F, -Cl, -Br, -I, $-O-C_{1...6}$-Alkyl, $-O-C_{6...14}$-Aryl, $-O(CO)R^6$, $-S-C_{1...6}$-Alkyl, $-S-C_{6...14}$Aryl, $-SOR^6$, $-SO_3H$, $-SO_2R^6$, $-OSO_2C_{1...6}$Alkyl, $-OSO_2C_{6...14}$Aryl, $-(CS)R^6$, -COOH, $-(CO)R^6$, mono-, bi- oder tricyclische gesättigte oder ein- oder mehrfach ungesättigte Carbocyclen mit 3 ...14 Ringgliedern, mono-, bi- oder tricyclische gesättigte oder ein- oder mehrfach ungesättigte Heterocyclen mit 5...15 Ringgliedern und 1...6 Heteroatomen, die vorzugsweise N, O und S sind, wobei die $C_{6...14}$Aryl-Gruppen und die eingeschlossenen carbocyclischen und heterocyclischen Substituenten ihrerseits ggf. ein- oder mehrfach mit $R^4$ substituiert sein können,

-carbo- oder heterocyclische gesättigte oder ein- oder mehrfach ungesättigte Spirocyclen mit 3...10 Ringgliedern, wobei heterocyclische Systeme 1...6 Heteroatome enthalten, die vorzugsweise N, O und S sind,

ggf. ein- oder mehrfach substituiert mit -OH, -SH, $-NH_2$, $-NHC_{1...6}$-Alkyl, $-N(C_{1...6}\text{-Alkyl})_2$, $-NHC_{6...14}Aryl)$, $-N(C_{6...14}Aryl)_2$, $-N(C_{1...6}Alkyl)(C_{6...14}Aryl)$, $-NHCOR^6$, $-NO_2$, -CN, -F, -Cl, -Br, -I, $-O-C_{1...6}$-Alkyl, $-O-C_{6...14}$-Aryl, $-O(CO)R^6$, $-S-C_{1...6}$-Alkyl, $-S-C_{6...14}$Aryl, $-SOR^6$, $-SO_3H$, $-SO_2R^6$, $-OSO_2C_{1...6}$Alkyl, $-OSO_2C_{6...14}$Aryl, $-(CS)R^6$, -COOH, $-(CO)R^6$, mono-, bi- oder tricyclische gesättigte oder ein- oder mehrfach ungesättigte Carbocyclen mit 3 ...14 Ringgliedern, mono-, bi- oder tricyclische gesättigte oder ein- oder mehrfach ungesättigte Heterocyclen mit 5...15 Ringgliedem und 1...6 Heteroatomen, die vorzugsweise N, O und S sind, wobei die $C_{6...14}$Aryl-Gruppen und die eingeschlossenen carbocyclischen und heterocyclischen Substituenten ihrerseits ggf, ein- oder mehrfach mit $R^4$ substituiert sein können,

**$R^2$, $R^3$** können Wasserstoff oder -OH sein, wobei mindestens einer von beiden Substituenten -OH sein muß;

**$R^4$** steht für

-H, -OH, -SH, $-NH_2$, $-NHC_{1...6}$-Alkyl, $-N(C_{1...6}\text{-Alkyl})_2$, $-NHC_{6...14}$Aryl, $-N(C_{6...14}Aryl)_2$, $-N(C_{1...6}Alkyl)(C_{6...14}Aryl)$, $-NHCOR^6$, $-NO_2$, -CN, -COOH, $-(CO)R^6$, $-(CS)R^6$, -F, -Cl, -Br, -I, $-O-C_{1...6}$-Alkyl, $-O-C_{6...14}$-Aryl, $-O(CO)R^6$, $-S-C_{1...6}$-Alkyl, $-S-C_{6...14}$Aryl, $-SOR^6$, $-SO_2R^6$;

**$R^5$** steht für

-mono-, bi- oder tricyclische gesättigte oder ein- oder mehrfach ungesättigte Carbocyclen mit 3 ... 14 Ringgliedern,

ggf. ein- oder mehrfach substituiert mit -OH, -SH, $-NH_2$, $-NHC_{1...6}$-Alkyl, $-N(C_{1...6}\text{-Alkyl})_2$, $-NHC_{6...14}$-Aryl, $-N(C_{6...14}\text{-Aryl})_2$, $-N(C_{1...6}\text{-Alkyl})(C_{6...14}\text{-Aryl})$, $-NHCOR^6$, $-NO_2$, -CN, -F, -Cl, -Br, -I, $-O-C_{1...6}$-Alkyl, $-O-C_{6...14}$-Aryl, $-O(CO)R^6$, $-S-C_{1...6}$-Alkyl, $-S-C_{6...14}$Aryl, $-SOR^6$, $-SO_3H$, $-SO_2R^6$, $-OSO_2C_{1...6}$-Alkyl, $-OSO_2C_{6...14}$-Aryl, $-(CS)R^6$, -COOH, $-(CO)R^6$, mono-, bi- oder tricyclische gesättigte oder ein- oder mehrfach ungesättigte Carbocyclen mit 3 ...14 Ringgliedem, mono-, bi- oder tricyclische gesättigte oder ein- oder mehrfach ungesättigte Heterocyclen mit 5...15 Ringgliedem und 1...6 Heteroatomen, die vorzugsweise N, O und S sind, wobei die $C_{6...14}$-Aryl-Gruppen und die eingeschlossenen carbocyclischen und heterocyclischen Substituenten ihrerseits ggf. ein- oder mehrfach mit $R^4$ substituiert sein können,

-mono-, bi- oder tricyclische gesättigte oder ein- oder mehrfach ungesättigte Heterocyclen mit 5... 15 Ringgliedern und 1...6 Heteroatomen, die vorzugsweise N, O und S sind,

ggf. ein- oder mehrfach substituiert mit -OH, -SH, $-NH_2$, $-NHC_{1...6}$-Alkyl, $-N(C_{1...6}\text{-Alkyl})_2$, $-NHC_{6...14}$-Aryl, $-N(C_{6...14}\text{-Aryl})_2$, $-N(C_{1...6}\text{-Alkyl})(C_{6...14}\text{-Aryl})$, $-NHCOR^6$, $-NO_2$, -CN, -F, -Cl, -Br, -I, $-O-C_{1...6}$-Alkyl, $-O-C_{6...14}$-Aryl, $-O(CO)R^6$, $-S-C_{1...6}$-Alkyl, $-S-C_{6...14}$-Aryl, $-SOR^6$, $-SO_3H$, $-SO_2R^6$, $-OSO_2C_{1...6}$-Alkyl, $-OSO_2C_{6...14}$-Aryl, $-(CS)R^6$, -COOH, $-(CO)R^6$, mono-, bi- oder tricyclische gesättigte oder ein- oder mehrfach ungesättigte Carbocyclen mit 3 ...14 Ringgliedem, mono-, bi- oder tricyclische gesättigte oder ein- oder mehrfach ungesättigte Heterocyclen mit 5...15 Ringgliedem und 1...6 Heteroatomen, die vorzugsweise N, O und S sind, wobei die $C_{6...14}$-Aryl-Gruppen und die eingeschlossenen carbocyclischen und heterocyclischen Substituenten ihrerseits ggf. ein- oder mehrfach mit $R^4$ substituiert sein können,

steht, wobei die Carbocyclen oder Heterocyclen mindestens einen Substituenten ausgewählt aus -F, -Cl, -Br und -I aufweisen,

**$R^6$** -H, $-NH_2$, $-NHC_{1...6}$-Alkyl, $-N(C_{1...6}\text{-Alkyl})_2$, $-NHC_{6...14}$-Aryl, $-N(C_{6...14}\text{-Aryl})_2$, $-N(C_{1...6}\text{-Alkyl})(C_{6...14}\text{-Aryl})$, $-O-C_{1...6}$-Alkyl, $-O-C_{6...14}$-Aryl, $-S-C_{1...6}$-Alkyl, $-S-C_{6...14}$-Aryl,
$-C_{1...12}$-Alkyl, geradkettig oder verzweigtkettig,
$-C_{1...12}$-Alkenyl, ein oder mehrfach ungesättigt, geradkettig oder verzweigtkettig,
-mono-, bi- oder tricyclische gesättigte oder ein- oder mehrfach ungesättigte Carbocyclen mit 3 ... 14 Ringgliedern,
-mono-, bi- oder tricyclische gesättigte oder ein- oder mehrfach ungesättigte Heterocyclen mit 5... 15 Ringgliedern und 1...6 Heteroatomen, die vorzugsweise N, O und S sind,

bedeuten,

**A** steht entweder für eine Bindung, oder für

$-(CH_2)_m-$, $-(CH_2)_m-(CH{=}CH)_n-(CH_2)_p-$, $-(CHOZ)_m-$, -(C=O)-, -(C=S)-, -(C=N-Z)-, -O-, -S-, -NZ-,

wobei m, p = 0 ... 3 und n = 0... 2 sind,

**Z** steht für

-H, oder
$-C_{1...12}$-Alkyl, geradkettig oder verzweigtkettig,
$-C_{1...12}$-Alkenyl, ein oder mehrfach ungesättigt, geradkettig oder verzweigtkettig,
-mono-, bi- oder tricyclische gesättigte oder ein- oder mehrfach ungesättigte Carbocyclen mit 3... 14 Ringgliedern,
-mono-, bi- oder tricyclische gesättigte oder ein- oder mehrfach ungesättigte Heterocyclen mit 5... 15 Ringgliedern und 1...6 Heteroatomen, die vorzugsweise N, O und S sind,

**B** kann entweder Kohlenstoff oder Schwefel sein, oder -(S=O)- bedeuten,

**D** kann Sauerstoff, Schwefel, $CH_2$ oder N-Z sein,
wobei **D** nur dann S oder $CH_2$ sein kann, wenn **B** Kohlenstoff bedeutet, und

**E** kann für eine Bindung stehen, oder aber für

$-(CH_2)_m-$, -O-, -S-, -(N-Z)-, wobei m und **Z** die bereits zuvor beschriebene Bedeutung besitzen.

2. Physiologisch verträgliche Salze der Verbindungen nach Formel 1 gemäß Anspruch 1, **gekennzeichnet durch** Neutralisation der Basen mit anorganischen oder organischen Säuren bzw. **durch** Neutralisation der Säuren mit anorganischen oder organischen Basen bzw. **durch** Quaternierung tertiärer Amine zu quaternären Ammoniumsalzen.

3. Verbindungen nach Formel 1 gemäß Anspruch 1 oder 2 mit einem asymmetrischen Kohlenstoffatom in der D-Form, der L-Form und D,L-Mischungen sowie im Falle mehrerer asymmetrischer Kohlenstoffatome die diastereomeren Formen.

4. Verbindungen nach Formel 1 gemäß einem der Ansprüche 1 bis 3 ausgewählt aus:

   N-(3,5-Dichlorpyridin-4-yl)-2-[1-(4-fluorbenzyl)-5-hydroxy-indol-3-yl]-2-oxo-acetamid und physiologisch verträglichen Salzen davon.

5. Verbindungen nach Formel 1 gemäß einem der Ansprüche 1 bis 3 ausgewählt aus einer der folgenden Verbindungen und physiologisch verträglichen Salzen davon:

   N-(3,5-Dichlorpyridin-4-yl)-2-[1-(4-fluorbenzyl)-5-hydroxy-indol-3-yl]-2-hydroxy-acetamid;

   N-(3,5-Dichlorpyridin-4-yl)-2-[1-(2,6-difluorbenzyl)-5-hydroxy-indol-3-yl]-2-oxo-acetamid;

   N-(3,5-Dichlorpyridin-4-yl)-2-[1-(3-nitrobenzyl)-5-hydroxy-indol-3-yl]-2-oxo-acetamid-Na-Salz;

   N-(3,5-Dichlorpyridin-4-yl)-2-(1-propyl-5-hydroxy-indol-3-yl)-2-oxo-acetamid;

   N-(3,5-Dichlorpyridin-4-yl)-2-(1-isopropyl-5-hydroxy-indol-3-yl)-2-oxo-acetamid;

   N-(3,5-Dichlorpyridin-4-yl)-2-(1-cyclopentylmethyl-5-hydroxy-indol-3-yl)-2-oxo-acetamid;

   N-(2,6-Dichlorphenyl)-2-[1-(4-fluorbenzyl)-5-hydroxy-indol-3-yl]-2-oxo-acetamid;

   N-(2,6-Dichlor-4-trifluormethyl-phenyl)-2-[1-(4-fluorbenzyl)-5-hydroxy-indol-3-yl]-2-oxo-acetamid;

   N-(2,6-Dichlor-4-trifluormethoxy-phenyl)-2-[1-(4-fluorbenzyl)-5-hydroxy-indol-3-yl]-2-oxo-acetamid;

   N-(3,5-Dichlorpyridin-4-yl)-2-[1-(4-fluorbenzyl)-6-hydroxy-indol-3-yl]-2-oxo-acetamid;

   N-(3,5-Dichlorpyridin-4 yl)-5-hydroxy-1-(4-methoxybenzyl)-indol-3-carbonsäureamid.

6. Verfahren zur Herstellung von Verbindungen nach Formel 1 gemäß einem der Ansprüche 1 bis 5, **gekennzeichnet dadurch, daß** Verbindungen gemäß Formel 1, für die $R^2$ oder $R^3$ bzw. $R^2$ und $R^3$ = $-O-R^7$ bedeuten, durch Abspaltung von $R^7$ in die erfindungsgemäßen Verbindungen überführt werden, wobei $R^7$ dabei für als Abgangsgruppe geeignete Substituenten steht.

7. Verfahren gemäß Anspruch 6, worin $R^7$ Alkyl-, Cycloalkyl-, Arylalkyl-, Aryl-, Heteroaryl-, Acyl-, Alkoxycarbonyl-, Aryloxycarbonyl-, Aminocarbonyl-, N-substituierte Aminocarbonyl-, Silyl-, Sulfonyl-Gruppen sowie Komplexbildner, wie zum Beispiel Verbindungen der Borsäure, der Phosphorsäure sowie kovalent oder koordinativ gebundene Metalle, wie Zink, Aluminium oder Kupfer bedeutet.

8. Verfahren zur Herstellung von Verbindungen nach Formel 1 gemäß einem der Ansprüche 1 bis 5, **gekennzeichnet dadurch, daß** Verbindungen der allgemeinen Formel 1, durch Umwandlungen der Teilstruktur:

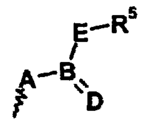

in andere erfindungsgemäße Verbindungen der Formel 1 überführt werden.

**9.** Verwendung der Verbindungen nach Formel 1 gemäß einem der Ansprüche 1 bis 5 als therapeutische Wirkstoffe zur Herstellung von Arzneimitteln zur Behandlung von Erkrankungen, bei denen die Hemmung von TNF a therapeutisch nützlich ist.

**10.** Verwendung der Verbindungen nach Formel 1 gemäß einem der Ansprüche 1 bis 5 als therapeutische Wirkstoffe zur Herstellung von Arzneimitteln zur Behandlung von Erkrankungen, bei denen die Hemmung der Phosphodiesterase 4 therapeutisch nützlich ist.

**11.** Verwendung der Verbindungen nach Formel 1 gemäß einem der Ansprüche 1 bis 5 als therapeutische Wirkstoffe zur Herstellung von Arzneimitteln zur Behandlung von Erkrankungen, die mit dem Wirken von Eosinophilen verbunden sind.

**12.** Verwendung der Verbindungen nach Formel 1 gemäß einem der Ansprüche 1 bis 5 als therapeutische Wirkstoffe zur Herstellung von Arzneimitteln zur Behandlung von chronisch obstruktiven Lungenerkrankungen (COPD).

**13.** Arzneimittel, enthaltend eine oder mehrere Verbindungen gemäß einem der Ansprüche 1 bis 5 neben üblichen physiologisch verträglichen Trägern und/oder Verdünnungsmitteln beziehungsweise Hilfsstoffen.

**14.** Verfahren zur Herstellung eines Arzneimittels nach Anspruch 13, **gekennzeichnet dadurch, daß** eine oder mehrere Verbindungen gemäß einem der Ansprüche 1 bis 5 mit gebräuchlichen pharmazeutischen Trägerstoffen und/oder Verdünnungsmitteln beziehungsweise sonstigen Hilfsstoffen zu pharmazeutischen Zubereitungen verarbeitet beziehungsweise in eine therapeutisch anwendbare Form gebracht werden.

**Claims**

**1.** Hydroxyindoles of the formula 1

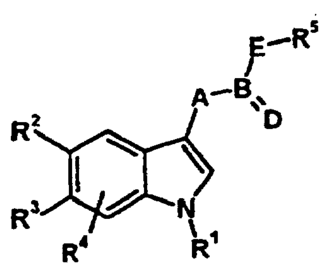

. 1

in which

**$R^1$** is

- $C_{1...12}$-alkyl, straight-chain or branched-chain,
  optionally mono- or polysubstituted by -OH, -SH, $-NH_2$, $-NHC_{1...6}$-alkyl, $-N(C_{1...6}\text{-alkyl})_2$, $-NHC_{6...14}$-aryl, $-N(C_{6...14}\text{-aryl})_2$, $-N(C_{1...6}$-alkyl) $(C_{6...14}$-aryl), $-NHCOR^6$, $-NO_2$, -CN, -F, -Cl, -Br, -I, $-O-C-_{1...6}$-alkyl, $-O-C_{6...14}$-aryl, $-O(CO)R^6$, $-S-C_{1...6}$-alkyl, $-S-C_{6...14}$-aryl, $-SOR^6$, $-SO_3H$, $-SO_2R^6$, $-OSO_2C_{1...6}$-alkyl, $-OSO_2C_{6...14}$-aryl,$-(CS)R^6$, -COOH, $-(CO)R^6$, mono-, bi- or tricyclic saturated or mono- or polyunsaturated carbocycles having 3...14 ring members, mono-, bi- or tricyclic saturated or mono- or polyunsaturated heterocycles having 5...15 ring members and 1...6 heteroatoms, which

are preferably N, O and S, where the $C_{6...14}$-aryl groups and the included carbocyclic and heterocyclic substituents for their part can optionally be mono- or polysubstituted by $R^4$,

- $C_{1...12}$-alkenyl, mono- or polyunsaturated, straight-chain or branched-chain,
  optionally mono- or polysubstituted by -OH, -SH, $-NH_2$, $-NHC_{1...6}$-alkyl, $-N(C_{1...6}\text{-alkyl})_2$, $-NHC_{6...14}$-aryl, $-N(C_{6...14}\text{-aryl})_2$, $-N(C_{1...6}$-alkyl$)(C_{6...14}$-aryl), $-NHCOR^6$, $-NO_2$, -CN, -F, -Cl, -Br, -I, $-O-C-_{1...6}$-alkyl, $-O-C_{6...14}$-aryl, $-O(CO)R^6$, $-S-C_{1...6}$-alkyl, $-S-C_{6...14}$-aryl, $-SOR^6$, $-SO_3H$, $-SO_2R^6$, $-OSO_2C_{1...6}$-alkyl, $-OSO_2C_{6...14}$-aryl, $-(CS)R^6$, -COOH, $-(CO)R^6$, mono-, bi- or tricyclic saturated or mono- or polyunsaturated carbocycles having 3...14 ring members, mono-, bi- or tricyclic saturated or mono- or polyunsaturated heterocycles having 5...15 ring members and 1...6 heteroatoms, which are preferably N, O and S, where the $C_{6...14}$-aryl groups and the included carbocyclic and heterocyclic substituents for their part can optionally be mono- or polysubstituted by $R^4$,

- mono-, bi- or tricyclic saturated or mono- or polyunsaturated carbocycles having 3...14 ring members,
  optionally mono- or polysubstituted by -OH, -SH, $-NH_2$, $-NHC_{1...6}$-alkyl, $-N(C_{1...6}\text{-alkyl})_2$, $-NHC_{6...14}$-aryl, $-N(C_{6...14}\text{-aryl})_2$, $-N(C_{1...6}$-alkyl$)$ $(C_{6...14}$-aryl), $-NHCOR^6$, $-NO_2$, -CN, -F, -Cl, -Br, -I, $-O-C-_{1...6}$-alkyl, $-O-C_{6...14}$-aryl, $-O(CO)R^6$, $-S-C_{1...6}$-alkyl, $-S-C_{6...14}$-aryl, $-SOR^6$, $-SO_3H$, $-SO_2R^6$, $-OSO_2C_{1...6}$-alkyl, $-OSO_2C_{6...14}$-aryl, $-(CS)R^6$, -COOH, $-(CO)R^6$, mono-, bi- or tricyclic saturated or mono- or polyunsaturated carbocycles having 3...14 ring members, mono-, bi- or tricyclic saturated or mono- or polyunsaturated heterocycles having 5...15 ring members and 1...6 heteroatoms, which are preferably N, O and S, where the $C_{6...14}$-aryl groups and the included carbocyclic and heterocyclic substituents for their part can optionally be mono- or polysubstituted by $R^4$,

- mono-, bi- or tricyclic saturated or mono- or polyunsaturated heterocycles having 5...15 ring members and 1...6 heteroatoms, which are preferably N, O and S,
  optionally mono- or polysubstituted by -OH, -SH, $-NH_2$, $-NHC_{1...6}$-alkyl, $-N(C_{1...6}\text{-alkyl})_2$, $-NHC_{6...14}$-aryl, $-N(C_{6...14}\text{-aryl})_2$, $-N(C_{1...6}$-alkyl$)$ $(C_{6...14}$-aryl), $-NHCOR^6$, $-NO_2$, -CN, -F, -Cl, -Br, -I, $-O-C-_{1...6}$-alkyl, $-O-C_{6...14}$-aryl, $-O(CO)R^6$, $-S-C_{1...6}$-alkyl, $-S-C_{6...14}$-aryl, $-SOR^6$, $-SO_3H$, $-SO_2R^6$, $-OSO_2C_{1...6}$-alkyl, $-OSO_2C_{6...14}$-aryl, $-(CS)R^6$, -COOH, $-(CO)R^6$, mono-, bi- or tricyclic saturated or mono- or polyunsaturated carbocycles having 3...14 ring members, mono-, bi- or tricyclic saturated or mono- or polyunsaturated heterocycles having 5...15 ring members and 1...6 heteroatoms, which are preferably N, O and S, where the $C_{6...14}$-aryl groups and the included carbocyclic and heterocyclic substituents for their part can optionally be mono- or polysubstituted by $R^4$,

- carbo- or heterocyclic saturated or mono- or polyunsaturated spirocycles having 3...10 ring members, where heterocyclic systems contain 1...6 heteroatoms, which are preferably N, O and S,
  optionally mono- or polysubstituted by -OH, -SH, $-NH_2$, $-NHC_{1...6}$alkyl, $-N(C_{1...6}\text{-alkyl})_2$, $-NHC_{6...14}$-aryl, $-N(C_{6...14}\text{-aryl})_2$, $-N(C_{1...6}$-alkyl$)$ $(C_{6...14}$-aryl), $-NHCOR^6$, $-NO_2$, -CN, -F, -Cl, -Br, -I, $-O-C-_{1...6}$-alkyl, $-O-C_{6...14}$-aryl, $-O(CO)R^6$, $-S-C_{1...6}$-alkyl, $-S-C_{6...14}$aryl, $-SOR^6$, $-SO_3H$, $-SO_2R^6$, $-OSO_2C_{1...6}$-alkyl, $-OSO_2C_{6...14}$-aryl, $-(CS)R^6$, -COOH, $-(CO)R^6$, mono-, bi- or tricyclic saturated or mono- or polyunsaturated carbocycles having 3...14 ring members, mono-, bi- or tricyclic saturated or mono- or polyunsaturated heterocycles having 5...15 ring members and 1...6 heteroatoms, which are preferably N, O and S, where the $C_{6...14}$-aryl groups and the included carbocyclic and heterocyclic substituents for their part can optionally be mono- or polysubstituted by $R^4$,

**$R^2$, $R^3$** can be hydrogen or -OH, where at least one of the two substituents must be -OH;

**$R^4$** is
-H, -OH, -SH, $-NH_2$, $-NHC_{1...6}$-alkyl, $-N(C_{1...6}\text{-alkyl})_2$, $-NHC_{6...14}$-aryl, $-N(C_{6...14}\text{-aryl})_2$, $-N(C_{1...6}$-alkyl$)$ $(C_{6...14}$-aryl), $-NHCOR^6$, $-NO_2$, -CN, -COOH, $-(CO)R^6$, $-(CS)R^6$, -F, -Cl, -Br, -I, $-O-C_{1...6}$-alkyl, $-O-C_{6...14}$-aryl, $-O(CO)R^6$, $-S-C_{1...6}$-alkyl, $-S-C_{6...14}$-aryl, $-SOR^6$, $-SO_2R^6$.

**$R^5$** is

- mono-, bi- or tricyclic saturated or mono- or polyunsaturated carbocycles having 3...14 ring members,
  mono- or polysubstituted by -F, -Cl, -Br, -I,

optionally mono- or polysubstituted by -OH, -SH, $-NH_2$, $-NHC_{1...6}$-alkyl, $-N(C_{1...6}\text{-alkyl})_2$, $-NHC_{6...14}$-aryl, $-N(C_{6...14}\text{-aryl})_2$, $-N(C_{1...6}\text{-alkyl})(C_{6...14}\text{-aryl})$, $-NHCOR^6$, $-NO_2$, -CN, $-O-C_{1...6}$-alkyl, $-O-C_{6...14}$-aryl, $-O(CO)R^6$, $-S-C_{1...6}$-alkyl, $-S-C_{6...14}$-aryl, $-SOR^6$, $-SO_3H$, $-SO_2R^6$, $-OSO_2C_{1...6}$-alkyl, $-OSO_2C_{6...14}$-aryl, $-(CS)R^6$, -COOH, $-(CO)R^6$, mono-, bi- or tricyclic saturated or mono- or polyunsaturated carbocycles having 3...14 ring members, mono-, bi- or tricyclic saturated or mono- or polyunsaturated heterocycles having 5...15 ring members and 1...6 heteroatoms, which are preferably N, O and S, where the $C_{6...14}$-aryl groups and the included carbocyclic and heterocyclic substituents for their part can optionally be mono- or polysubstituted by $R^4$,

- mono-, bi- or tricyclic saturated or mono- or polyunsaturated heterocycles having 5...15 ring members and 1...6 heteroatoms, which are preferably N, O and S,

  mono- or polysubstituted by -F, -Cl, -Br, -I,

  optionally mono- or polysubstituted by -OH, -SH, $-NH_2$, $-NHC_{1...6}$-alkyl, $-N(C_{1...6}\text{-alkyl})_2$, $-NHC_{6...14}$-aryl, $-N(C_{6...14}\text{-aryl})_2$, $-N(C_{1...6}\text{-alkyl})\ (C_{6...14}\text{-aryl})$, $-NHCOR^6$, $-NO_2$, -CN, $-O-C_{1...6}$-alkyl, $-O-C_{6...14}$-aryl, $-O(CO)R^6$, $-S-C_{1...6}$-alkyl, $-S-C_{6...14}$-aryl, $-SOR^6$, $-SO_3H$, $-SO_2R^6$, $-OSO_2C_{1...6}$-alkyl, $-OSO_2C_{6...14}$-aryl, $-(CS)R^6$, -COOH, $-(CO)R^6$, mono-, bi- or tricyclic saturated or mono- or polyunsaturated carbocycles having 3...14 ring members, mono-, bi- or tricyclic saturated or mono- or polyunsaturated heterocycles having 5...15 ring members and 1...6 heteroatoms, which are preferably N, O and S, where the $C_{6...14}$-aryl groups and the included carbocyclic and heterocyclic substituents for their part can optionally be mono- or polysubstituted by $R^4$,

**$R^6$** can be

-H, $-NH_2$, $-NHC_{1...6}$-alkyl, $-N(C_{1...6}\text{-alkyl})_2$, $-NHC_{6...14}$-aryl, $-N(C_{6...14}\text{-aryl})_2$, $-N(C_{1...6}\text{-alkyl})\ (C_{6...14}\text{-aryl})$, $-O-C_{1...6}$-alkyl, $-O-C_{6...14}$-aryl, $-S-C_{1...6}$-alkyl, $-S-C_{6...14}$-aryl,
$-C_{1...12}$-alkyl, straight-chain or branched-chain,
$-C_{1...12}$-alkenyl, mono- or polyunsaturated, straight-chain or branched-chain,
-mono-, bi- or tricyclic saturated or mono- or polyunsaturated carbocycles having 3...14 ring members
-mono-, bi- or tricyclic saturated or mono- or polyunsaturated heterocycles having 5...15 ring members and 1...6 heteroatoms, which are preferably N, O and S;

**A** is either a bond, or

$-CH_2)_m-$, $-(CH_2)_m-(CH=CH)_n-(CH_2)_p-$, $-(CHOZ)_m-$, -(C=O)-, -(C=S)-, -(C=N-Z)-, -O-, -S-, -NZ-,

where m, p = 0...3 and n = 0...2 and

**z** is

-H, or
where m, p = 0...3 and n = 0...2 and
$-C_{1..12}$-alkyl, straight-chain or branched-chain,
$-C_{1...12}$-alkenyl, mono- or polyunsaturated, straight-chain or branched-chain,
-mono-, bi- or tricyclic saturated or mono- or polyunsaturated carbocycles having 3...14 ring members,
-mono-, bi- or tricyclic saturated or mono- or polyunsaturated heterocycles having 5...15 ring members and 1...6 heteroatoms, which are preferably N, O and S;

**B** can either be carbon or sulphur, or -(S=O)-;

**D** can be oxygen, sulphur, $CH_2$ or N-Z,
where **D** can only be S or $CH_2$ if **B** is carbon; and

**E** can be a bond, or else

$-(CH_2)_m-$, -O-, -S-, -(N-Z)-, where m and **Z** have the meaning already described beforehand.

**2.** Physiologically tolerable salts of the compounds as shown in formula 1 according to Claim 1, **characterized by** neutralization of the bases with inorganic or organic acids or by neutralization of the acids with inorganic or organic bases or by quaternization of tertiary amines to give quaternary ammonium salts.

**3.** Compounds as shown in formula 1 according to Claim 1 or 2 having an asymmetric carbon atom in the D form, the L form and D,L mixtures, and, in the case of a number of asymmetric carbon atoms, the diastereomeric forms.

**4.** Compounds as shown in formula 1 according to one of Claims 1 to 3, selected from:

N-(3,5-dichloropyridin-4-yl)-2-[1-(4-fluorobenzyl)-5-hydroxyindol-3-yl]-2-oxoacetamide; and physiologically tolerable salts thereof.

**5.** Compounds as shown in formula 1 according to one of Claims 1 to 3 selected from one of the following compounds and physiologically tolerable salts thereof:

N-(3,5-dichloropyridin-4-yl)-2-[1-(4-fluorobenzyl)-5-hydroxyindol-3-yl]-2-hydroxyacetamide;

N-(3,5-dichloropyridin-4-yl)-2-[1-(2,6-difluorobenzyl)-5-hydroxyindol-3-yl]-2-oxoacetamide;

*N*-(3,5-dichloropyridin-4-yl)-2-[1-(3-nitrobenzyl)-5-hydroxyindol-3-yl]-2-oxoacetamide Na salt;

N-(3,5-dichloropyridin-4-yl)-2-(1-propyl-5-hydroxy-indol-3-yl)-2-oxoacetamide;

N-(3,5-dichloropyridin-4-yl)-2-(1-isopropyl-5-hydroxyindol-3-yl)-2-oxoacetamide;

N-(3,5-dichloropyridin-4-yl)-2-(1-cyclopentylmethyl-5-hydroxyindol-3-yl)-2-oxoacetamide;

N-(2,6-dichlorophenyl)-2-[1-(4-fluorobenzyl)-5-hydroxyindol-3-yl]-2-oxoacetamide;

N-(2,6-dichloro-4-trifluoromethylphenyl)-2-[1-(4-fluorobenzyl)-5-hydroxyindol-3-yl]-2-oxoacetamide;

N-(2,6-dichloro-4-trifluoromethoxyphenyl)-2-[1-(4-fluorobenzyl)-5-hydroxyindol-3-yl]-2-oxoacetamide;

N-(3,5-dichloropyridin-4-yl)-2-[1-(4-fluorobenzyl)-6-hydroxyindol-3-yl]-2-oxoacetamide;

N-(3,5-dichloropyridin-4-yl)-5-hydroxy-1-(4-methoxybenzyl)indole-3-carboxamide.

**6.** Process for the preparation of compounds as shown in formula 1 according to one of Claims 1 to 5, **characterized in that** compounds as shown in formula 1, for which $R^2$ or $R^3$ or $R^2$ and $R^3$ = -O-$R^7$, are converted into the compounds according to the invention by removal of $R^7$, where $R^7$ in this case is a substituent suitable as a leaving group.

**7.** Process according to Claim 6, in which $R^7$ is alkyl, cycloalkyl, arylalkyl, aryl, heteroaryl, acyl, alkoxycarbonyl, aryloxycarbonyl, aminocarbonyl, N-substituted aminocarbonyl, silyl or sulphonyl groups, and complexing agents, such as, for example, compounds of boric acid, phosphoric acid and covalently or coordinatively bonded metals, such as zinc, aluminium or copper.

**8.** Process for the preparation of compounds as shown in formula 1 according to one of Claims 1 to 5, **characterized in that** compounds of the general formula 1 are converted by means of conversions of the sub-structure:

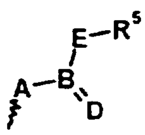

into other compounds of the formula 1 according to the invention.

**9.** Use of the compounds as shown in formula 1 according to one of Claims 1 to 5 as therapeutic active compounds for the production of medicaments for the treatment of disorders in which the inhibition of TNFα is therapeutically beneficial.

**10.** Use of the compounds as shown in formula 1 according to one of Claims 1 to 5 as therapeutic active compounds for the production of medicaments for the treatment of disorders in which the inhibition of phosphodiesterase 4 is therapeutically beneficial.

**11.** Use of the compounds as shown in formula 1 according to one of Claims 1 to 5 as therapeutic active compounds for the production of medicaments for the treatment of disorders which are connected with the action of eosinophils.

**12.** Use of the compounds as shown in formula 1 according to one of Claims 1 to 5 as therapeutic active compounds for the production of medicaments for the treatment of chronic obstructive pulmonary diseases (COPD).

**13.** Medicaments comprising one or more compounds according to one of Claims 1 to 5 in addition to customary physiologically tolerable carriers and/or diluents or auxiliaries.

**14.** Process for the production of a medicament according to Claim 13, **characterized in that** one or more compounds according to Claims 1 to 5 are processed to give pharmaceutical preparations or brought into a therapeutically administrable form using customary pharmaceutical carriers and/or diluents or other auxiliaries.

**Revendications**

**1.** Hydroxyindole de formule 1

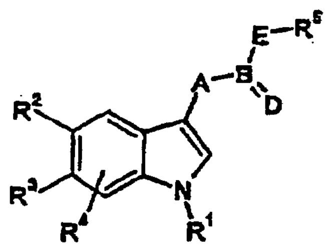

(1)

dans laquelle

$R^1$ signifie

- un alkyle en $C_1$ à $C_{12}$, à chaîne linéaire ou ramifiée, le cas échéant monosubstitué ou polysubstitué par -OH, -SH, $-NH_2$, -NH-alkyle en $C_1$ à $C_6$, -N-(alkyle en $C_1$ à $C_6)_2$, -NH-aryle en $C_6$ à $C_{14}$, -N-(aryle en $C_6$ à $C_{14})_2$, -N-(alkyle en $C_1$ à $C_6$) (aryle en $C_6$ à $C_{14}$), $-NHCOR^6$, $-NO_2$, -CN, -F, -Cl, -Br, -I, -O-alkyle en $C_1$ à $C_6$, -O-aryle en $C_6$ à $C_{14}$, $-O(CO)R^6$, -S-alkyle en $C_1$ à $C_6$, -S-aryle en $C_6$ à $C_{14}$, $-SOR^6$, $-SO_3H$, $-SO_2R^6$, $-OSO_2$-alkyle en $C_1$ à $C_6$, $-OSO_2$-aryle en $C_6$ à $C_{14}$, $-(CS)R^6$, -COOH, $-(CO)R^6$, des carbocycles monoinsaturés ou polyinsaturés ou monocycliques, bicycliques ou tricycliques saturés avec 3 à 14 chaînons, des hétérocycles monoinsaturés ou polyinsaturés ou monocycliques, bicycliques ou tricycliques saturés avec 5 à 15 chaînons et 1 à 6 hétéroatomes, qui sont de préférence N, O et S, où les groupes $C_6$ à $C_{14}$ aryle et les substituants carbocycliques et hétérocycliques inclus peuvent, quant à eux, être monosubstitués ou polysubstitués par $R^4$,

- un alcényle en $C_2$ à $C_{12}$, monoinsaturé ou polyinsaturé, à chaîne linéaire ou ramifiée, le cas échéant monosubstitué ou polysubstitué par -OH, -SH, $-NH_2$, -NH-alkyle en $C_1$ à $C_6$, -N-(alkyle en $C_1$ à $C_6)_2$, -NH-aryle en $C_6$ à $C_{14}$, -N-(aryle en $C_6$ à $C_{14})_2$, -N-(alkyle en $C_1$ à $C_6$) (aryle en $C_6$ à $C_{14}$), $-NHCOR^6$, $-NO_2$, -CN, -F, -Cl, -Br, -I, -O-alkyle en $C_1$ à $C_6$, -O-aryle en $C_6$ à $C_{14}$, $-O(CO)R^6$, -S-alkyle en $C_1$ à $C_6$, -S- aryle

en $C_6$ à $C_{14}$, -$SOR^6$, -$SO_3H$, -$SO_2R^6$, -$OSO_2$-alkyle $C_1$ à $C_6$, -$OSO_2$-aryle $C_6$ à $C_{14}$, -(CS)$R^6$, -COOH, -(CO)$R^6$, des carbocycles monoinsaturés ou polyinsaturés ou monocycliques, bicycliques ou tricycliques saturés avec 3 à 14 chaînons, des hétérocycles monoinsaturés ou polyinsaturés ou monocycliques, bicycliques ou tricycliques saturés avec 5 à 15 chaînons et 1 à 6 hétéroatomes, qui sont de préférence N, O et S, où les groupes aryle en $C_6$ à $C_{14}$ et les substituants carbocycliques et hétérocycliques inclus peuvent, quant à eux, être monosubstitués ou polysubstitués par $R^4$,

- des carbocycles monoinsaturés ou polyinsaturés ou monocycliques, bicycliques ou tricycliques saturés avec 3 à 14 chaînons,

  le cas échéant monosubstitués ou polysubstitués par -OH, -SH, -$NH_2$, -NH-alkyle en $C_1$ à $C_6$, -N-(alkyle $C_1$ à $C_6$)$_2$, -NH-aryle en $C_6$ en $C_{14}$, -N-(aryle $C_6$ en $C_{14}$)$_2$, -N-(alkyle en $C_1$ à $C_6$)(aryle $C_6$ à $C_{14}$), -$NHCOR^6$, -$NO_2$, -CN, -F, -Cl, -Br, -I, -O- alkyle en $C_1$ à $C_6$, -O-aryle en $C_6$ à $C_{14}$, -O(CO)$R^6$, -S-alkyle $C_1$ à $C_6$, -S-aryle en $C_6$ à $C_{14}$, -$SOR^6$, -$SO_3H$, -$SO_2R^6$, -$OSO_2$-alkyle $C_1$ à $C_6$, -$OSO_2$-aryle en $C_6$ à $C_{14}$, -(CS) $R^6$, -COOH, -(CO)$R^6$, des carbocycles monoinsaturés ou polyinsaturés ou monocycliques, bicycliques ou tricycliques saturés avec 3 à 14 chaînons, des hétérocycles monoinsaturés ou polyinsaturés ou monocycliques, bicycliques ou tricycliques saturés avec 5 à 15 chaînons et 1 à 6 hétéroatomes, qui sont de préférence N, O et S, où les groupes aryle en $C_6$ à $C_{14}$ et les substituants carbocycliques et hétérocycliques inclus peuvent, quant à eux, être monosubstitués ou polysubstitués par $R^4$,

- des hétérocycles monoinsaturés ou polyinsaturés ou monocycliques, bicycliques ou tricycliques saturés avec 5 à 15 chaînons et 1 à 6 hétéroatomes, qui sont de préférence N, O et S,

  le cas échéant monosubstitués ou polysubstitués par -OH, -SH, -$NH_2$, -NH-alkyle en $C_1$ à $C_6$, -N-(alkyle $C_1$ à $C_6$)$_2$, -NH-aryle $C_6$ à $C_{14}$, -N-(aryle en $C_6$ à $C_{14}$)$_2$, -N-(alkyle en $C_1$ à $C_6$)(aryle en $C_6$ à $C_{14}$), -$NHCOR^6$, $NO_2$, -CN, -F, -Cl, -Br, -I, -O-alkyle en $C_1$ à $C_6$, -O-aryle en $C_6$ à $C_{14}$, -O(CO)$R^6$, -S-alkyle en $C_1$ à $C_6$, -S-aryle en $C_6$ à $C_{14}$, -$SOR^6$, -$SO_3H$, -$SO_2R^6$, -$OSO_2$-alkyle en $C_1$ à $C_6$ -$OSO_2$-aryle en $C_6$ à $C_{14}$, -(CS)$R^6$, -COOH, -(CO)$R^6$, des carbocycles monoinsaturés ou polyinsaturés ou monocycliques, bicycliques ou tricycliques saturés avec 3 à 14 chaînons, des hétérocycles monoinsaturés ou polyinsaturés ou monocycliques, bicycliques ou tricycliques saturés avec 5 à 15 chaînons et 1 à 6 hétéroatomes, qui sont de préférence N, O et S, où les groupes aryle en $C_6$ à $C_{14}$ et les substituants carbocycliques et hétérocycliques inclus peuvent, quant à eux, être monosubstitués ou polysubstitués par $R^4$,

- des cycles spiraniques carbocycliques et hétérocycliques saturés ou monoinsaturés ou polyinsaturés avec 3 à 10 chaînons, alors que les systèmes hétérocycliques contiennent 1 à 6 hétéroatomes, qui sont de préférence N, O et S,

  le cas échéant monosubstitués ou polysubstitués par -OH, -SH, -$NH_2$, -NH-alkyle en $C_1$ à $C_6$, -N-(alkyle en $C_1$ à $C_6$)$_2$, -NH-aryle en $C_6$ à $C_{14}$, -N-(aryle en $C_6$ à $C_{14}$)$_2$, -N-(alkyle en $C_1$ à $C_6$)(aryle en $C_6$ à $C_{14}$), -$NHCOR^6$, -$NO_2$, -CN, -F, -Cl, -Br, -I, -O-alkyle $C_1$ à $C_6$, -O-aryle $C_6$ à $C_{14}$, -O(CO)$R^6$, -S-alkyle en $C_1$ à $C_6$, -S- aryle en $C_6$ à $C_{14}$, -$SOR^6$, -$SO_3H$, -$SO_2R^6$, -$OSO_2$-alkyle en $C_1$ à $C_6$, -$OSO_2$-aryle en $C_6$ à $C_{14}$, -(CS)$R^6$, -COOH, -(CO)$R^6$, des carbocycles monoinsaturés ou polyinsaturés ou monocycliques, bicycliques ou tricycliques saturés avec 3 à 14 chaînons, des hétérocycles monoinsaturés ou polyinsaturés ou monocycliques, bicycliques ou tricycliques saturés avec 5 à 15 chaînons et 1 à 6 hétéroatomes, qui sont de préférence N, O et S, où les groupes aryle en $C_6$ à $C_{14}$ et les substituants carbocycliques et hétérocycliques inclus peuvent, quant à eux, être monosubstitués ou polysubstitués par $R^4$,

$R^2$, $R^3$ peuvent être de l'hydrogène ou -OH, sachant qu'au moins un des deux substituants doit être -OH ;

$R^4$ signifie

-H, -OH, -SH, -$NH_2$, -NH-alkyle en $C_1$ à $C_6$, -N-(alkyle en $C_1$ à $C_6$)$_2$, -NH-aryle en $C_6$ à $C_{14}$, -N-(aryle en $C_6$ à $C_{14}$)$_2$, -N-(alkyle en $C_1$ à $C_6$)(aryle en $C_6$ à $C_{14}$), -$NHCOR^6$, -$NO_2$, -CN, -COOH, -(CO)$R^6$, -(CS)$R^6$, -F, -Cl, -Br, -I, -O-alkyle en $C_1$ à $C_6$, -O-aryle en $C_6$ à $C_{14}$, -O(CO)$R^6$, -S-alkyle en $C_1$ à $C_6$, -S-aryle en $C_6$ à $C_{14}$, -$SOR^6$, -$SO_2R^6$;

$R^5$ signifie
des carbocycles monoinsaturés ou polyinsaturés ou mcnocycliques, bicycliques ou tricycliques saturés avec 3 à 14 chaînons,
monosubstitués ou polysubstitués par F, -Cl, -Br, -I,
le cas échéant monosubstitués ou polysubstitués par -OH, -SH, -$NH_2$, -NH-alkyle $C_1$ à $C_6$, -N-(alkyle en

$C_1$ à $C_6)_2$, -NH-aryle en $C_6$ à $C_{14}$, -N-(aryle en $C_6$ à $C_{14})_2$, -N-(alkyle en $C_1$ à $C_6$)(aryle en $C_6$ à $C_{14}$), -NHCOR$^6$, $NO_2$, -CN, -O-alkyle en $C_1$ à $C_6$, -O-aryle en $C_6$ à $C_{14}$, -O(CO)R$^6$, -S- alkyle en $C_1$ à $C_6$, -S-aryle en $C_6$ à $C_{14}$, -SOR$^6$, -$SO_3$H, -$SO_2$R$^6$ -$OSO_2$-alkyle en $C_1$ à $C_6$, -$OSO_2$-aryle en $C_6$ à $C_{14}$, -(CS)R$^6$, -COOH, -(CO)R$^6$, des carbocycles monoinsaturés ou polyinsaturés ou monocycliques, bicycliques ou tricycliques saturés avec 3 à 14 chaînons, des hétérocycles monoinsaturés ou polyinsaturés ou monocycliques, bicycliques ou tricycliques saturés avec 5 à 15 chaînons et 1 à 6 hétéroatomes, qui sont de préférence N, O et S, où les groupes aryle en $C_6$ à $C_{14}$ et les substituants carbocycliques et hétérocycliques inclus peuvent, quant à eux, être monosubstitués ou polysubstitués par R$^4$,

- des hétérocycles monoinsaturés ou polyinsaturés ou monocycliques, bicycliques ou tricycliques saturés avec 5 à 15 chaînons et 1 à 6 hétéroatomes, qui sont de préférence N, O et S,
  monosubstitués ou polysubstitués par -F, -Cl, -Br, -I,
  le cas échéant monosubstitués ou polysubstitués par -OH, -SH, -$NH_2$, -NH-alkyle en $C_1$ à $C_6$, -N-(alkyle $C_1$ à $C_6)_2$, -NH-aryle en $C_6$ à $C_{14}$, -N-(aryle en $C_6$ à $C_{14})_2$, -N-(alkyle en $C_1$ à $C_6$)(aryle en $C_6$ à $C_{14}$), -NHCOR$^6$, -$NO_2$, -CN, -O-alkyle en $C_1$ à $C_6$, -O-aryle en $C_6$ à $C_{14}$, -O(CO)R$^6$, -S-alkyle en $C_1$ à $C_6$, -S-aryle en $C_6$ à $C_{14}$, -SOR$^6$, -$SO_3$H, -$SO_2$R$^6$, -$OSO_2$-alkyle en $C_1$ à $C_6$, -$OSO_2$-aryle en $C_6$ à $C_{14}$, -(CS)R$^6$, -COOH, -(CO)R$^6$, des carbocycles monoinsaturés ou polyinsaturés ou monocycliques, bicycliques ou tricycliques saturés avec 3 à 14 chaînons, des hétérocycles monoinsaturés ou polyinsaturés ou monocycliques, bicycliques ou tricycliques saturés avec 5 à 15 chaînons et 1 à 6 hétéroatomes, qui sont de préférence N, O et S, où les groupes aryle en $C_6$ à $C_{14}$ et les substituants carbocycliques et hétérocycliques inclus peuvent, quant à eux, être le cas échéant monosubstitués ou polysubstitués par R$^4$,

R$^6$ peut signifier

- H, -$NH_2$, -NH-alkyle en $C_1$ à $C_6$, -N-(alkyle en $C_1$ à $C_6)_2$, -NH- aryle en $C_6$ à $C_{14}$, -N-(aryle en $C_6$ à $C_{14})_2$, -N-(alkyle en $C_1$ à $C_6$)(aryle en $C_6$ à $C_{14}$), -O-alkyle en $C_1$ à $C_6$, -O-aryle en $C_6$ à $C_{14}$, -S-alkyle en $C_1$ à $C_6$, -S-aryle en $C_6$ à $C_{14}$,
- un alkyle en $C_1$ à $C_{12}$, à chaîne linéaire ou ramifiée,
- un alcényle en $C_2$ à $C_{12}$, monoinsaturé ou polyinsaturé, à chaîne linéaire ou ramifiée,
- des carbocycles monoinsaturés ou polyinsaturés ou monocycliques, bicycliques ou tricycliques saturés avec 3 à 14 chaînons,
- des hétérocycles monoinsaturés ou polyinsaturés ou monocycliques, bicycliques ou tricycliques saturés avec 5 à 15 chaînons et 1 à 6 hétéroatomes, qui sont de préférence N, O et S,

A signifie soit une liaison soit

-$(CH_2)_m$,-,-$(CH_2)_m$-(CH=CH)$_n$-$(CH_2)_p$-, -$(CHOZ)_m$-, -(C=O)-, -(C=S)-, -(C=N=Z)-, -O-, -S-, -NZ-,

où m,p = 0 à 3 et n = 0 à 2,

Z signifie

- H, ou
- un alkyle en $C_1$ à $C_{12}$, à chaîne linéaire ou ramifiée,
- un alcényle en $C_2$ à $C_{12}$, monoinsaturé ou polyinsaturé, à chaîne linéaire ou ramifiée,
- des carbocycles monoinsaturés ou polyinsaturés ou monocycliques, bicycliques ou tricycliques saturés avec 3 à 14 chaînons,
- des hétérocycles monoinsaturés ou polyinsaturés ou monocycliques, bicycliques ou tricycliques saturés avec 5 à 15 chaînons et 1 à 6 hétéroatomes, qui sont de préférence N, O et S,

B peut être soit du carbone ou du soufre, ou signifier -(S=O)-,

D peut être soit de l'oxygène, du soufre, $CH_2$ ou N-Z,
dont D ne peut être S ou $CH_2$, seulement si B signifie du carbone, et

E peut signifier une liaison ou bien
-$(CH_2)_m$-, -O-, -S-, -(N-Z)-, où m et Z ont la signification décrite auparavant.

2. Sels physiologiquement compatibles des composés d'après la formule 1 selon la revendication 1, **caractérisés par** la neutralisation des bases avec des acides organiques ou inorganiques ou par la neutralisation des acides avec des bases organiques ou inorganiques ou par la quaternisation d'amines tertiaires en sels d'ammonium quaternaires.

3. Composés d'après la formule 1 selon la revendication 1 ou 2 avec un atome de carbone asymétrique dans la forme D, la forme L et les mélanges D,L ainsi que les formes diastéréomères dans les cas de plusieurs atomes de carbone asymétriques.

4. Composés d'après la formule 1 selon l'une quelconque des revendications 1 à 3 sélectionnés parmi :

   N-(3,5-dichloropyridine-4-yl)-2-[1-(4-fluorobenzyle)-5-hydroxy-indol-3-yl]-2-oxo-acétamide et sels compatibles physiologiquement de cela.

5. Composés d'après la formule 1 selon l'une quelconque des revendications 1 à 3 sélectionnés parmi un des composés suivants et ses sels physiologiquement compatibles :

   N-(3,5-dichloropyridine-4-yl)-2-[1-(4-fluorobenzyle)-5-hydroxy-indole-3-yl]-2-hydroxy-acétamide ;

   N-(3,5-dichloropyridine-4-yl)-2-[1-(2,6-difluorobenzyle)-5-hydroxy-indole-3-yl]-2-oxo-acétamide ;

   sel de sodium du N-(3,5-dichloropyridine-4-yl)-2-[1-(3-nitrobenzyle)-5-hydroxy-indole-3-yl]-2-oxo-acétamide ;

   N-(3,5-dichloropyridine-4-yl)-2-(1-propyle-5-hydroxy-indole-3-yl)-2-oxo-acétamide ;

   N-(3,5-dichloropyridine-4-yl)-2-(1-isopropyle-5-hydroxy-indole-3-yl)-2-oxo-acétamide ;

   N-(3,5-dichloropyridine-4-yl)-2-(1-cyclopentylmethyle-5-hydroxy-indole-3-yl)-2-oxo-acétamide ;

   N-(2,6-dichlorophényle)-2-[1-(4-fluorobenzyle)-5-hydroxy-indole-3-yl]-2-oxo-acétamide ;

   N-(2,6-dichloro-4-trifluorméthyle-phényle)-2-[1-(4-fluorobenzyle)-5-hydroxy-indole-3-yl]-2-oxo-acétamide

   N-(2,6-dichloro-4-trifluorméthoxy-phényle)-2-[1-(4-fluorobenzyle)-5-hydroxy-indole-3-yl]-2-oxo-acétamide ;

   N-(3,5-dichloropyridine-4-yl)-2-[1-(4-fluorobenzyle)-6-hydroxy-indole-3-yl]-2-oxo-acétamide ;

   Amide de l'acide N-(3,5-dichloropyridine-4-yl)-5-hydroxy-1-(4-méthoxybenzyle)-indole-3-Carboxylique.

6. Procédé pour la préparation des composés d'après la formule 1 selon l'une quelconque des revendications 1 à 5, **caractérisé en ce que** les composés d'après la formule 1 pour lesquels $R^2$ ou $R^3$ ou $R^2$ et $R^3$ = O-$R^7$ sont transformés en composés selon l'invention par la dissociation de $R^7$, sachant que $R^7$ représente des substituants appropriés comme groupes partants.

7. Procédé selon la revendication 6, dans lequel $R^7$ signifie des groupes alkyle, cycloalkyle, arylalkyle, aryle, hétéroaryle, acyle, alcoxycarbonyle, aryloxycarbonyle, aminocarbonyle, aminocarbonyle N-substitué, silyle et sulfonyle ainsi que des agents de complexation, comme par exemple des composés d'acide borique, phosphorique ou de métaux covalents ou coordinants comme le zinc, l'aluminium ou le cuivre.

8. Procédé pour produire des composés d'après la formule 1 selon l'une quelconque des revendications 1 à 5, **caractérisé en ce que** les composés de la formule générale 1 sont transformés en d'autres composés selon l'invention de la formule 1 par transformations de la structure partielle :

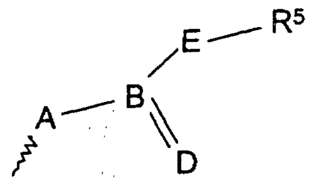

**9.** Utilisation des composés d'après la formule 1 selon l'une quelconque des revendications 1 à 5 comme substances thérapeutiques pour préparer des médicaments pour le traitement d'affections pour lesquelles l'inhibition du FNT a (TNF a) est utile d'un point de vue thérapeutique.

**10.** Utilisation des composés d'après la formule 1 selon l'une quelconque des revendications 1 à 5 comme substances thérapeutiques pour préparer des médicaments pour le traitement d'affections pour lesquelles l'inhibition de la phosphodiesterase 4 est utile d'un point de vue thérapeutique.

**11.** Utilisation des composés d'après la formule 1 selon l'une quelconque des revendications 1 à 5 comme substances thérapeutiques pour préparer des médicaments pour le traitement d'affections, qui sont liées à l'action des éosinophiles.

**12.** Utilisation des composés d'après la formule 1 selon l'une quelconque des revendications 1 à 5 comme des substances thérapeutiques pour préparer des médicaments pour le traitement des maladies pulmonaires obstructives chroniques (MPOC).

**13.** Médicament contenant un ou plusieurs composés selon l'une quelconque des revendications 1 à.5 en plus de supports physiologiquement compatibles habituels et/ou des diluants ou agents auxiliaires.

**14.** Procédé pour fabriquer un médicament selon la revendication 13, **caractérisé en ce qu'**un ou plusieurs composés, selon l'une quelconque des revendications 1 à 5, sont transformés avec des supports pharmaceutiques usuels et/ou diluants ou autres agents auxiliaires en des préparations pharmaceutiques ou convertis dans une forme applicable d'un point vue thérapeutique.